# EUROPEAN PATENT SPECIFICATION

(11) **EP 3 928 323 B1**
(45) Date of publication and mention of the grant of the patent: **21.10.2026**
(21) Application number: 20711755.7
(22) Date of filing: 21.02.2020
(51) Int. Cl.: G16H 20/17, G16H 40/60

(54) **METHODS, SYSTEMS AND DEVICES FOR A MEDICAMENT DOSE CALCULATOR**
VERFAHREN, SYSTEME UND VORRICHTUNGEN FÜR EINEN MEDIKAMENTENDOSISRECHNER
PROCÉDÉS, SYSTÈMES ET DISPOSITIFS POUR UN CALCULATEUR DE DOSE DE MÉDICAMENT

(30) Priority: 21.02.2019 US 201962808735 P
(43) Date of publication of application: 29.12.2021
(73) Proprietor: Medtronic Minimed, Inc., Northridge, CA 91325-1219 (US)
(72) Inventor: MENSINGER, Michael, San Diego, California 92127 (US); BENKE, Jasper, San Diego, California 92127 (US); SAINT, Sean, San Diego, California 92127 (US); COHEN, Eric, San Diego, California 92127 (US); PRYOR, Jack, San Diego, California 92127 (US); HAMPAPURAM, Haripriyan, San Diego, California 92127 (US)
(74) Representative: Zimmermann & Partner Patentanwälte mbB
(86) International application number: PCT/US2020/019360
(87) International publication number: WO 2020/172628

(56) References cited:
- WO-A1-2013/102158
- US-A1- 2012 232 520
- US-A1- 2016 117 481
- US-A1- 2018 353 698

## Description

### TECHNICAL FIELD

This patent document relates to medicine administering and tracking systems, devices, and processes.

### BACKGROUND

A typical insulin bolus (dose) calculator works by evaluating a diabetic person's (also referred to as "patient") current blood glucose level (BG), the insulin in their body from previous doses (e.g., insulin on board or IOB), and the number of grams of carbohydrates ("carbs") the user is or recently has been eating. From these values and the patient's pre-set clinical parameters, a conventional dose calculator computes an estimated amount of fast-acting insulin to take, based on clinically-validated established equations.

While BG can be measured directly and IOB can be calculated explicitly based on recent insulin doses, the grams of carbs must be manually estimated by the user. This leads to inaccuracy, difficulty in training, and some users being unwilling or unable to use a dose calculator. Yet, for patients, using a dose calculator is much more accurate than the patient roughly estimating or guessing at the proper dose; as such, a dose calculator can contribute to better glycemic control, better safety, and better health. For these reasons, it is desirable for a dose calculator to be used even by people who cannot or will not estimate carbs.

US 2016/0117481 A1 relates to subcutaneous outpatient management.

US 2012/0232520 A1 relates to a multi-function analyte monitor device and methods of use.

US 2018/0353698 A1 relates to intelligent medication delivery systems and methods.

### SUMMARY

The invention is defined in the dependent claims. Preferred embodiments are defined by the dependent claims.

Disclosed are alternatives to the traditional dose calculator that provides estimating carbs but with adaptations of simpler methods of estimating meals for augmenting a dose calculator.

While in some implementations, for example, a carb-estimating augmented dose calculator can provide meal estimation options that may have lower precision than the cumbersome task of strict carb-counting, but for some users may be sufficient and may be a vast improvement over their current method of guessing or mentally estimating doses. In some implementations of the of the carb-estimating augmented dose calculator, the carb-estimating augmented dose calculator adapts itself to perform simpler methods with certain users (e.g., newly diagnosed patients who need to start simple) and increases complexity as these users grow more familiar and experienced with their therapy.

In addition to simplified meal estimation, more streamlined methods of viewing and obtaining dose recommendations and logging meals are disclosed. These are intended to decrease burden on the user, increase compliance and use of the system, and promote training to a proper diabetes management regimen as prescribed by their physician.

The disclosed methods, systems and devices include a smart medicine delivery pen ("smart pen") or other insulin delivery device that automatically logs doses in a software application comprising the dose calculator, which the software application can be resident on the smart pen and/or on a companion device, like a smartphone, smartwatch, or other computing device in wireless communication with the smart pen, for example, via a wireless Bluetooth connection. Examples of systems and devices that can implement the disclosed methods, systems and devices are described in PCT Patent Application Publication No. WO2019/0753 52A1, entitled "INTELLIGENT MEDICATION DELIVERY SYSTEMS AND METHODS FOR DOSE RECOMMENDATION AND MANAGEMENT,".

In some embodiments in accordance with the present technology, a method for adjusting an insulin dose size by fixed-dose titration on an injection pen device in wireless communication with a mobile communication device includes receiving a first glucose measurement of a patient user of the injection pen device prior to consumption of a meal; determining a first dose size of insulin to be recommended for administration to the patient user based on consumption of the meal, wherein the determined first dose size of insulin is selected from a predefined insulin amount that corresponds to (i) a meal type, or (ii) the meal type and a meal size of the meal type; presenting to the patient user, via a display on at least one of the injection pen device or the mobile communication device, the recommended first dose size of insulin to be administered to the patient user; receiving a second glucose measurement of the patient user within a predefined time period after consumption of the meal by the patient user; determining a second dose size of insulin to be recommended for administration to the patient user for correcting the second glucose measurement to be within a target glucose level range; and presenting to the patient user, via the display, the recommended second dose size of insulin to be administered to the patient user.

In some embodiments in accordance with the present technology, a system for administering a medicine using a fixed-dose titration protocol includes an injection pen device including a dose setting mechanism to set a dose of a medicine contained in a medicine cartridge that is to be dispensed by the injection pen device, a dispensing mechanism to dispense the medicine according to the set dose, and an electronics unit including a processor, a memory comprising instructions executable by the processor, and a wireless transmitter, the processor of the injection pen device configured to generate dose data associated with a dispensing event of a dose of the medicine dispensed from the injection pen device and time data associated with the dispensing event, and to wirelessly transmit the dose data, wherein the medicine includes insulin, wherein the injection pen device is in wireless communication with a mobile communication device that includes a data processing unit including a processor and memory to receive and process the dose data, and wherein the mobile communication device includes a software application program product comprising a non-transitory computer-readable storage medium having instructions, which when executed by the processor of the data processing unit, cause the mobile communication device to determine a recommended one or more fixed-dose sizes of the insulin based on (i) health data, including first glucose level of a patient user of the injection pen device that is measured prior to consumption of a meal and a second glucose level of the patient user that is measured within a predefined time period after consumption of the meal, and (ii) meal data, including a meal type of the meal and a meal size of the meal.

### BRIEF DESCRIPTION OF THE DRAWINGS

FIG. 1A shows a diagram of an example embodiment of an intelligent medicine administration system in accordance with the present technology.
FIG. 1B shows a diagram of an example embodiment of a pen device in communication with a companion device of the intelligent medicine administration system of FIG. 1A.
FIG. 1C shows a block diagram of an example embodiment of the companion device of the intelligent medicine administering system of FIG. 1A.
FIG. 1D shows a schematic illustration of an example embodiment of the pen device shown in FIG. 1B.
FIGS. 2A and 2B show diagrams of example embodiments of a method for improving meal dose size estimates and adjusting an insulin dose size by fixed-dose titration, in accordance with the disclosed technology.
FIGS. 3A and 3B show diagrams of example embodiments of a method for executing an initial setup of a simplified or streamlined dose calculator, in accordance with the disclosed technology.
FIGS. 4A and 4B show diagrams of example embodiments of a method for executing an initial setup of a dose calculator and recommending an insulin dose based on a total daily insulin dose, in accordance with the disclosed technology.
FIG. 5 shows a diagram of an example embodiment of a method for calculating a correction meal recommendation without a defined insulin-to-carb ratio, in accordance with the disclosed technology.
FIG. 6 shows a diagram of an example embodiment of a method for autonomous insulin dose recording without user interaction for an insulin dose calculator, in accordance with the disclosed technology.

### DETAILED DESCRIPTION

Various diseases and medical conditions, such as diabetes, require a patient to self-administer doses of a fluid medication. Typically, when administering a fluid medication, the appropriate dose amount is set and dispensed by the patient using a syringe, a pen, or a pump. For example, self-administered medicaments or medicine include insulin used to treat diabetes, Follistim^{®} used to treat infertility, or other injectable medicines such as Humira^{®}, Enbrel^{®}, Lovenox^{®} and Ovidrel^{®}, or others.

A medicament pen is a device that can be used to inject a quantity of a medicine (e.g., single or multiple boluses or doses of the medicine) into a user's body, where more than one dose can be stored in a medicine cartridge contained in the pen device. Pens offer the benefit of simplicity over other methods of delivery, such as syringe or pump-based methods. For example, syringes typically require more steps to deliver a dose, and pumps typically are more complicated to use and require a constant tether to the patient. However, previously there has not been an automated way to track and communicate the doses given with the pen in a simple, effective and reliable manner. In addition, it can be difficult to know how much to dose with the pen, when to dose, or if the patient dosed at all.

As with the dosing of any medication, it is sometimes hard for a patient to remember if a dose has been given. For this reason, for example, pill reminders have been developed where the patient places the medication for the day in a cup labeled with that day. Once they take their medication, there is no question it has been taken because the pills are no longer in the cup. Yet, there are no widely acceptable solutions that address this problem for injection-based therapies. Therefore, without simple, effective and reliable ways of tracking medicine doses, particularly for managing lifelong or chronic conditions like diabetes, patients may easily miss a dose or administer an incorrect dose (e.g., under-dose or over-dose) of their medicine which may result in serious, dangerous consequences to their health.

In addition to the challenges of tracking doses, calculating the right dose at the right time or under the right conditions is a widespread problem for patients of chronic conditions requiring daily dosing of medicine. Conventional dose calculators for administering insulin for Type I and Type II diabetes typically require manual estimation of carbohydrates ("carbs") at mealtime. For some users, carb-counting and estimating may be too difficult, and some users may not utilize the dose calculator due to the manual work and number of steps required to do so, e.g., taking out one's smartphone, opening up an app, manually typing calculator inputs, etc.

Moreover, conventional bolus or basal insulin dose calculators may operate solely using a pre-set, fixed dosing parameters, as defined by the patient user's physician, that inform the dose calculator. Primary variables of insulin dose are just the user's analyte levels, food intake, and insulin on board. Yet, there are countless other factors that affect the user's glucose and insulin responses, some of which can be incorporated into refined dosing parameters.

Disclosed are systems, devices and methods for providing alternatives to the traditional dose calculator that provides estimating carbs but with adaptations of simpler methods of estimating meals for augmenting a dose calculator. In some aspects, the disclosed systems, devices and methods that provide a fixed-dose, meal estimation, and/or touchless dose calculator module for automated or semi-automated medicine dose recommendations for patient health management by the patient and their caregivers using medicine injection devices.

In some embodiments, the disclosed dose calculator module is embodied on a software application ("app") resident on (i) a patient user's device, which can include a medicine injection device (also referred to as the "pen" or "pen device") and/or (ii) the patient's "companion" device (e.g., such as a smartphone, smartwatch, or wearable communication device), which is in data communication with the pen device, and where one or both of which are able to detect and record dose sizes dialed on the pen device and delivered, including the capability of distinguishing between a priming dose and a therapy dose. Communication between the pen device and the companion device provides the ability for dose tracking, logging, calculating, recommending and/or communicating of dose data with a user (e.g., patient user, health care provider (HCP) and/or caregiver), and other advantages of the intelligent medicine administration system. For example, each bolus that is dispensed by the pen device can be automatically logged and communicated to the companion device. In some embodiments, the dose calculator module, or sub-modules thereof, can be resident on a computer system or communication network accessible via the Internet (referred to as 'the cloud') that includes one or more remote computational processing devices (e.g., servers in the cloud).

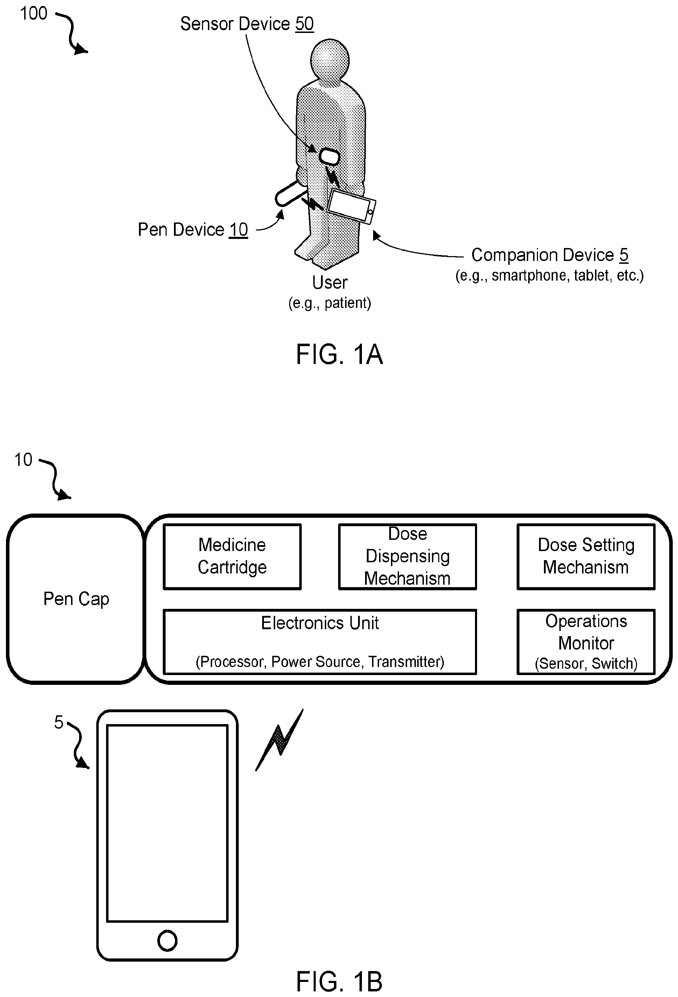

FIG. 1A shows a diagram of an example embodiment of an intelligent medicine administering system 100 in accordance with the present technology. The system 100 includes a pen device 10 in wireless communication with a mobile computing and communication device 5 of a patient user, also referred to as the user's companion device. The pen device 10 is operable to select, set and/or dispense a dose of the medicine for dispensing. In some implementations, the companion device 5 includes a smartphone, tablet, and/or wearable computing device, such as a smartwatch, smartglasses, etc. In some implementations, the companion device 5 is in communication with other computing devices, such as a laptop and/or desktop computer, a smart television, or network-based server computer. The companion device 5 includes an app associated with the pen device 10 of the intelligent medicine administering system 100, which can monitor and/or control functionalities of the pen device 10 and to provide the dose calculator module that can calculate and recommend a dose of the medicine for the patient user to administer using the pen device 10.

The companion device 5 can be used to obtain, process and/or display contextual data that can be used to relate to the patient user's health condition, including the condition for which the pen device 10 is used to treat. In an illustrative example, the companion device 5 is operable to track the patient user's location; the patient user's physical activity including step count, movement distance and/or intensity, estimated calories burned, and/or activity duration; and/or the patient user's interaction pattern with the companion device 5. The app associated with the system 100 can aggregate and process the contextual data to generate decision support outputs to guide and aid the patient user in using the pen device 10 and/or managing their behavior to promote better health outcomes in treating his/her health condition.

In some embodiments, the system 100 includes a sensor device 50 to monitor one or more health metrics of the patient user. Examples of health metric data monitored by the sensor device 50 include analytes, such as glucose, heart rate, blood pressure, user movement, or other. In some implementations, the sensor device 50 is a wearable sensor device such as a continuous glucose monitor (CGM) to obtain transcutaneous or blood glucose measurements that are processed to produce continuous glucose values. For example, the continuous glucose monitor can include a glucose processing module implemented on a stand-alone display device and/or implemented on the companion device 5, which processes, stores and displays the continuous glucose values for the patient user.

FIG. 1B shows a diagram of an example embodiment of the pen device 10 of the intelligent medicine administering system 100. The pen device 10 is structured to have a body which contains the medicine cartridge (e.g., which can be replaceable). The pen device 10 is structured to include a dose dispensing mechanism to dispense (e.g., deliver) the medicine contained in the medicine cartridge out of the pen device 10; a dose setting mechanism to select and/or set the dose to be dispensed; an operations monitoring mechanism to determine that the pen device 10 is being operated and/or to monitor the operation of the dose being dispensed (e.g., such as a switch and/or sensor, or an encoder); and an electronics unit that can include a processor, a memory, a battery or other power source, and a transmitter. In some embodiments, for example, the pen device 10 includes a display providing a user interface that displays output data (e.g., dialed and/or dispensed dose information, the recommended dose, or other) to a user of the pen device 10, and in some embodiments can input data from the user.

The pen device 10 is configured in communication with a user's mobile computing and communication device 5, e.g., such as the user's smartphone, tablet, and/or wearable computing device, such as a smartwatch, smartglasses, etc., and/or a user's laptop and/or desktop computer, a smart television, or network-based server computer.

In some implementations of the system 100, for example, to use the pen device 10, the user first dials up a dose using a dose knob. The dose knob of the pen device 10 can be included as part of the dose setting mechanism and/or the dose dispensing mechanism. For example, the dose may be adjusted up or down prior to administration of the dose. When the user applies a force against a dose dispensing button (e.g., presses against the dose dispensing button that is caused to protrude outward from the pen's body upon dialing the dose using the dose knob), a pushing component (e.g., also referred to as a 'plunger') of the dose dispensing mechanism is depressed against an abutment of the medicine cartridge loaded in the pen device 10 to cause the pen device 10 to begin to dispense the medicine, in which the quantity dispensed is in accordance with that set by the dose setting mechanism. In such implementations, the operations monitoring mechanism of the pen device 10 will begin to sense movement of a rotating component or shaft that drives the plunger, for example, in which the movement is sensed through an encoder. In some examples, the encoder can be configured to sense the rotation of a component that is coupled to the drive shaft, and as the drive shaft rotates the plunger moves linearly; and therefore by sensing rotation of the component, the movement of the drive shaft and the plunger is sensed. Movement of the encoder may be detected as data processed by a processor of the electronics unit of the pen device 10, which can be used to measure the dose. In some implementations, the processor can then store the size of the dose along with a time stamp for that dose. In some implementations, the pen device 10 can then transmit the dose and related information to the companion device 5. In such implementations when the dose is transmitted, the data associated with the particular transmitted dose is marked in the memory of the pen device 10 as transmitted. In such implementations if the dose was not yet transmitted to the companion device 5, then the data associated with the dose will be transmitted at the next time a successful communication link between the pen device 10 and the companion device 5 is established.

The operations monitor mechanism of the pen device 10 can include a sensor that can utilize any method of sensing rotary or linear movement. Non-limiting examples of such sensors include rotary and linear encoders, Hall effect and other magnetic based sensors, linearly variable displacement transducers, or any other appropriate method of sensing known in the art.

The dose dispensing mechanism of the pen device 10 can include a manually powered mechanism or a motorized mechanism. In either case, a force (e.g., either produced by the patient or by an electrically-powered motor) pushes on the plunger of the dose dispensing mechanism to in turn force a receiving plunger of the medicament vial or cartridge to deliver the specific amount of the medicament. In some implementations, for example, the dose dispensing mechanism can be adjusted to deliver the dose over a different period of time. In one example, the dose dispensing mechanism can be operated such that the plunger is pushed in by an adjustable tension spring or change the speed of the motor to inject the dose over a time frame (e.g., 1 s, 5 s or other) to aid in reducing the pain of dosing. In one example, the dose dispensing mechanism can be operated over a much longer period of time, e.g., to better match the dynamics of carbohydrates, which can be like an extended bolus with a pump.

The software application (app) of the pen device 10 and/or companion device 5 can provide a user interface displayable on a display of the pen device 10 and/or the companion device 5 to allow the user to manage his/her health related data. In some implementations, for example, the companion device 5 can be configured to control some functionalities of the pen device 10. In some implementations, for example, the companion device 5 includes the user's existing smartphone, tablet, or wearable computing device. In some implementations, for example, the companion device 5 is an independent portable device that the user may carry on his/her person. In one example embodiments of an independent portable companion device 5, the companion device 5 includes a data processing unit, wireless communication unit to allow the device to communicate with the pen device 10, and a display unit.

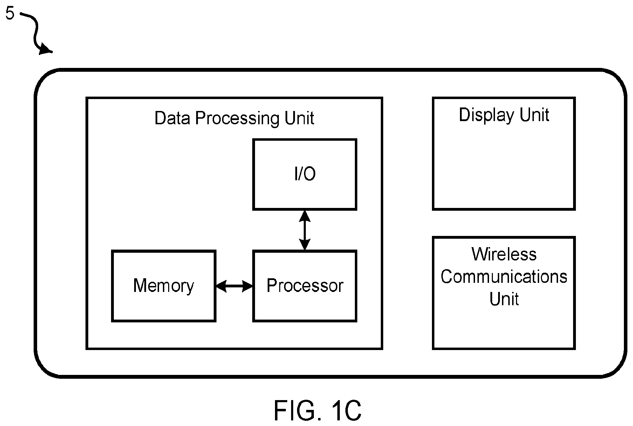

FIG. 1C shows a block diagram of an example embodiment of the companion device 5 of the intelligent medicine administration system 100. The data processing unit of the companion device 5 includes a processor to process data, a memory in communication with the processor to store data, and an input/output unit (I/O) to interface the processor and/or memory to other modules, units or devices of the companion device 5 or external devices. For example, the processor can include a central processing unit (CPU) or a microcontroller unit (MCU). For example, the memory can include and store processor-executable code, which when executed by the processor, configures the data processing unit to perform various operations, e.g., such as receiving information, commands, and/or data, processing information and data, and transmitting or providing information/data to another device. In some implementations, the data processing unit can transmit raw or processed data to a computer system or communication network accessible via the Internet ('the cloud') that includes one or more remote computational processing devices (e.g., servers in the cloud). To support various functions of the data processing unit, the memory can store information and data, such as instructions, software, values, images, and other data processed or referenced by the processor. For example, various types of Random Access Memory (RAM) devices, Read Only Memory (ROM) devices, Flash Memory devices, and other suitable storage media can be used to implement storage functions of the memory unit. The I/O of the data processing unit can interface the data processing unit with the wireless communications unit to utilize various types of wired or wireless interfaces compatible with typical data communication standards, for example, which can be used in communications of the data processing unit with other devices such as the pen device 10, via a wireless transmitter/receiver (Tx/Rx) unit, e.g., including, but not limited to, Bluetooth, Bluetooth low energy, Zigbee, IEEE 802.11, Wireless Local Area Network (WLAN), Wireless Personal Area Network (WPAN), Wireless Wide Area Network (WWAN), WiMAX, IEEE 802.16 (Worldwide Interoperability for Microwave Access (WiMAX)), 3G/4G/LTE cellular communication methods, NFC (Near Field Communication), and parallel interfaces. The I/O of the data processing unit can also interface with other external interfaces, sources of data storage, and/or visual or audio display devices, etc. to retrieve and transfer data and information that can be processed by the processor, stored in the memory unit, or exhibited on an output unit of the companion device 5 or an external device. For example, a display unit of the companion device 5 can be configured to be in data communication with the data processing unit, e.g., via the I/O, to provide a visual display, an audio display, and/or other sensory display that produces the user interface of the software application of the disclosed technology for health management. In some examples, the display unit can include various types of screen displays, speakers, or printing interfaces, e.g., including but not limited to, light emitting diode (LED), or liquid crystal display (LCD) monitor or screen, cathode ray tube (CRT) as a visual display; audio signal transducer apparatuses as an audio display; and/or toner, liquid inkjet, solid ink, dye sublimation, inkless (e.g., such as thermal or UV) printing apparatuses, etc.

In various operations of the disclosed intelligent medicine administration system, for example, when a dosing event (e.g., an amount of fluid is dispensed from the pen device 10), a time stamp associated with the dispensing is referenced is recorded by the processing unit of the pen device 10 (e.g., stored in the memory of the pen device 10). For example, the time stamp may be the current time or a time where a count-up timer is used. When the dose information is eventually transmitted to the companion device 5, the time stamp and/or a 'time-since-dose' parameter is transmitted by the pen device 10 and received by the companion device 5 and stored in the memory of the data processing unit of the companion device 5. In some implementations, for example, the time of the dose can be determined without the pen having to know the current time. This can simplify operation and setup of the pen device 10. In some implementations, for example, a user time is initialized on the pen device 10 from the companion device 5, in which the user time is used for dose time tracking. Using the system 100, the companion device 5 can know the time of the dose relative to the current time.

Once the companion device 5 receives the dose related information (e.g., which can include the time information and dose setting and/or dispensing information, and other information about the pen device 10 related to the dosing event), the companion device 5 stores the dose related information in memory, e.g., which can include among a list of doses or dosing events. For example, via the software application's user interface, the companion device 5 allows the patient to browse a list of previous doses, to view an estimate of current medicament active in the patient's body ("medicament on board") based on calculations performed by a medicine calculation module of the software application, and/or to utilize a dose calculation module of the software application to assist the patient regarding dose setting information on the size of the next dose to be delivered. For example, the patient could enter carbohydrates to be eaten, current blood sugar, and the companion device 5 would already know insulin on board. Using these parameters a suggested medicine dose (e.g., such as insulin dose), calculated by the dose calculation module, may be determined. In some implementations, for example, the companion device 5 can also allow the patient to manually enter boluses into the pen device 10 or another medicine delivery device. This would be useful if the patient was forced to use a syringe, or if the battery in the pen device 10 was depleted.

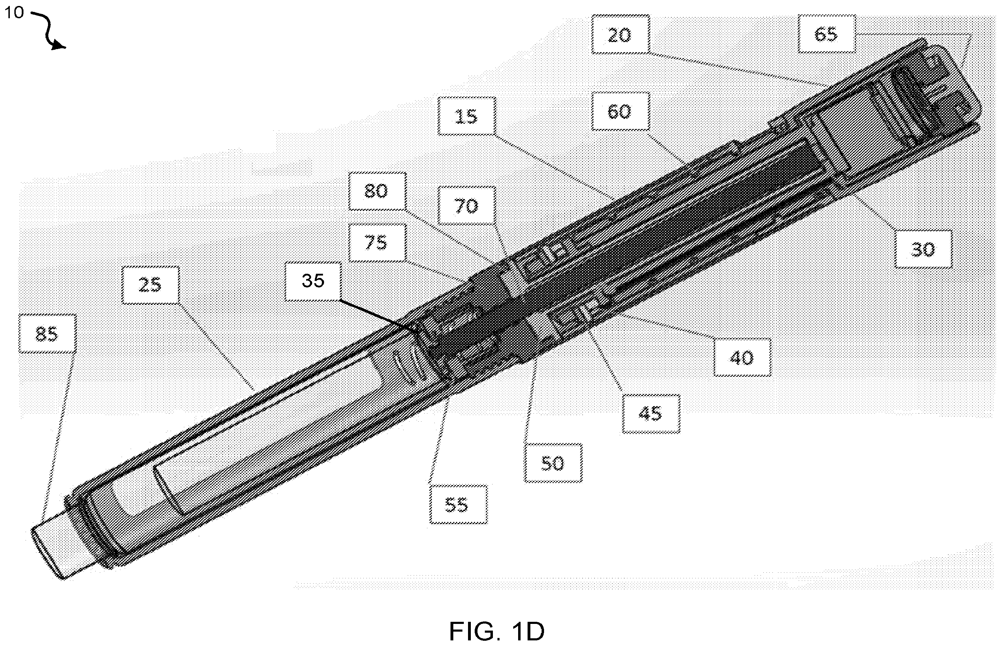

FIG. 1D shows a schematic illustration of an example embodiment of the pen device 10. The example shown in FIG. 1D illustrates the structural arrangement and interaction of the example modular units and mechanisms depicted in FIG. 1B for various operations of the pen device 10. As shown in FIG. 1D, the pen device 10 includes a mechanism to actuate a force to cause a displacement of a piston which resides within a medicament vial or cartridge 85. The displacement of the piston of the medicament vial 85 forces a volume of the medicament (that is proportional to the displacement of the piston) out of the vial 85, e.g., allowing it to be injected into a patient. The vial 85 is held within a medicament housing 25 of the pen device 10. The medicament housing 25 attaches to a main body housing 15 of the pen device 10, which includes a dose setting and dispensing mechanism and electronics unit of the pen device 10. In some embodiments, for example, the medicament housing 25 and the main body housing 15 may be a singular housing structure. The medicament housing 25 is structured to include a chamber to hold and/or encase the medicament vial 85 within the housing 25 of the pen device 10. The pen device 10 can also include a detachable pen cap (not shown) to cover an end of the pen device 10 that exposes a needle assembly (not shown) of the pen device 10 to disburse the medicine out of the pen device 10 when dispensed from the vial 85. The pen device 10 can include a vial spring 35, which provides a force on a screw retractor 55 to push the medicament vial 85 into the medicament housing 25 to ensure good dose accuracy. The pen device 10 includes a dose knob 20 attached to or included as part of the housing 15, where the dose knob is coupled to the housing by a non-self-locking thread 60. In some embodiments, for example, an electronics housing 30 may reside within the dose knob 20, in which the electronics housing 30 contains the electronics unit of the pen device 10. The dose setting mechanism includes a dose knob 20. When the dose knob 20 is rotated into or out of the housing 15 to adjust the dose, the electronics housing 30 does not turn. However, when translational or axial force is placed to the dose button 65 (e.g., in which resides the electronics housing), a catch structure component is engaged to lock the electronics housing 30 and dose knob 20 together, forcing them to rotate together as the pair travel back into the housing 15 upon actuation of the dose dispensing mechanism to apply force to the dose knob 20 to cause dispensing of the set dose. The rotation of the dose knob 20, e.g., which can be via the electronics housing 30, rotates a shaft 50 (e.g., which can be configured as a bi-directional clutch shaft). The shaft 50 in turn rotates a dose screw 70 which rides within a nut 75 which is fixed to the housing 15. This rotation causes the dose screw 70 to extend out of the housing 15 causing an injection of medicament. In some embodiments, for example, the dose dispensing mechanism can include a friction-causing structure 80, e.g., which can be coupled to the exemplary bi-directional clutch shaft 50 to present a frictionous surface (i.e., a surface that provides friction) to make contact with the nut 75 or housing body 15 or other internal structure of the dose dispensing mechanism, which acts from the bi-directional clutch shaft 50 to the housing 15 or nut 75 to prevent rotation of the shaft 50 while the dose setting mechanism is being adjusted via turning of the dose knob 20, but also allowing the friction to be overcome during the dose dispensing operation. In addition, by overcoming friction in the opposite direction the dose screw 70 may be driven back into the housing 15 and prepared for a new cartridge of medicament to be loaded. In some embodiments, for example, the pen device 10 includes a screw retractor component 55 that is axially fixed to the housing but rotationally free. The screw retractor component 55 is operable to be bent in to "grab" the non-circular cross section of the dose screw 70 allowing it to be rotated relative to the housing 15 and driven back into the housing 15. In some implementations, for example, the components of the pen device 10 could be manufactured by injection molding, machining or other similar process. In embodiments including the bi-directional clutch shaft, for example, the pen device 10 is capable of allowing retraction of the lead screw, and repeatability of operation of the dose dispensing mechanism.

In some embodiments, the sensor unit of the pen device 10 includes a rotational encoder, for example, between the dose knob 20 (e.g., which can be coupled to the jack screw) and the housing 15, and in electrical communication with the electronics unit contained in the electronics housing 30. The encoder is included in a sensor unit to determine the quantity of the dose set by the dose setting mechanism, and/or, the quantity of the dose dispensed by the dose dispensing mechanism. In some implementations, for example, the encoder can be configured in the pen device 10 to determine the dispensed dose by detecting rotation of the lead screw which is correlated with displacement of the pusher foot which is correlated with displacement of the receiving plunger in the vial 85, which in turn is correlated with dispensed insulin. In some embodiments, for example, the encoder can include two flat plates with contacts in between them. The plates are aligned perpendicular to the axis of the device. For one plate, a contact plate 40 is rotationally fixed to the jack screw, e.g., which can be via the electronics housing 30; and for the other plate, a code wheel 45 is rotationally fixed to the housing 15. In implementations, for example, when relative motion occurs between these two plates during dosing, the relative motion is measured and transmitted to the data processing and communications unit for processing, storage and/or transmission to the companion device 5.

In some embodiments of the pen device 10, for example, the dose setting and dispensing mechanism may include a mechanism in which the dose screw 70 is comprised of an elongate nut which screws in and out of the housing to provide dosing. The nut component in the previous described embodiment (e.g., nut 75) can include a separate screw structure; whereas in this embodiment of the dose screw, the nut component is part of the dose screw including exterior threads and is coupled to the housing 15. When the exemplary bi-directional clutch shaft 50 provides rotation, it operates on the jack screw, e.g., in which the dosing nut in this case threading it out of the housing.

The example embodiments and implementations of the pen device 10 and companion device 5 are described for facilitating understanding of some implementations of the various embodiments of the dose calculator module in a system, a device and as a method. While the disclosed embodiments described herein are primarily based on diabetes management systems and methods involving insulin pen and glucose monitoring devices to facilitate understanding of the underlying concepts, it is understood that the disclosed embodiments can also include treatment of other health conditions using other medications by the pen device and/or monitoring of other analytes by sensor devices.

### Fixed-Dose and Meal Size Estimation in a Dose Calculator

Typically users estimate grams of carbs in a meal and enter this into a bolus dose calculator. Yet, conventional dose calculators may require the user to account for several parameters, which can confuse users or burden them in a manner that results in their non-use of a dose calculator for insulin bolus dosing. As such, the integration into a dose calculator of alternative simpler methods are described below, including fixed-dose therapy and meal size estimation therapy techniques in accordance with the disclosed technology.

With respect to the disclosed techniques, fixed-dose therapy refers to techniques for determining a set dose of insulin for diabetes patients, where a single dose size of insulin may be prescribed for every meal (not necessarily including a corrective dose). A single dose size may also be called a "fixed dose." For example, for a fixed-dose therapy, a predefined, individual dose size for any meal (e.g., each meal like breakfast, lunch, dinner) may be referred to as a fixed dose, whereas a predefined, individual dose size for a particular meal or meal category may be referred to as a "fixed-meal dose." Both can be implemented similarly. For example, with all meal doses equal, the user may occasionally not need to select which meal is being eaten, or all pre-set meal doses could simply be set equal by the physician.

For meal-size estimation therapy, the process works much the same except the user additionally selects a relative meal size for the meal being eaten (for example lunch, small; or breakfast, large). In this case, each combination of meal type and size has a pre-defined dose size that is displayed or summed with the current BG correction dose as well. In the example of small/medium/large breakfast/lunch/dinner/snack this would represent twelve different preset dose sizes.

### Fixed-Dose Therapy

In some embodiments, the disclosed methods, devices and systems include a fixed-dose insulin bolus dose calculator module for improving the functioning of a diabetes management system. The fixed-dose therapy dose calculator module can be implemented via a software application operable on a computing device, such as the companion device 5 (e.g., smartphone, tablet smartwatch, smartglasses, etc.), and/or a medical device, such as the medicament delivery device 10 (e.g., insulin pen). The software application can be configured as a standalone software application for implementing the meal-type fixed-dose therapy dose calculator module or as an integrated feature of a software application directed to blood glucose level monitoring, insulin delivery, or other diabetes management application. The fixed-dose therapy calculator is configured to determine a pre-set dose size of insulin for the patient to administer in association with a meal to be eaten, that is being eaten, or has recently been eaten. For example, in example implementations of the fixed-dose therapy dose calculator, the user would select which meal (e.g., breakfast, lunch, dinner, snack) is being eaten. Each meal has a pre-set dose size defined for the particular patient user, and this pre-set dose value would be displayed on a user interface of the device implementing the fixed-dose therapy dose calculator. In some implementations, if BG data (e.g., from a manual entry or a connected finger-stick meter or continuous glucose monitor) was available, the fixed-dose therapy dose calculator may also determine a correction dose for the patient user in this instance, and the sum of the correction dose and the pre-set dose size would be displayed as the complete dose to administer.

### Fixed-Meal-Dose Therapy

In some embodiments, the disclosed methods, devices and systems include a meal-type and size fixed-dose insulin bolus dose calculator module for improving the functioning of a diabetes management system. The meal-type and size fixed-dose therapy dose calculator module can be implemented via a software application operable on a computing device, such as the companion device 5 (e.g., smartphone, tablet smartwatch, smartglasses, etc.), and/or a medical device, such as the medicament delivery device 10 (e.g., insulin pen). The software application can be configured as a standalone software application for implementing the meal-type and size fixed-dose therapy dose calculator module or as an integrated feature of a software application directed to blood glucose level monitoring, insulin delivery, or other diabetes management application. The meal-type and size fixed-dose therapy calculator is configured to determine a pre-set dose size of insulin for the patient to administer in association with a type of meal and size of the meal to be eaten, that is being eaten, or has recently been eaten. For example, in example implementations of the meal-type and size fixed-dose therapy dose calculator, the user would select which meal (e.g., breakfast, lunch, dinner, snack) to be / is being / has been eaten. Similarly, the meal-type and size fixed-dose calculator can present a display where the user would select a meal size (e.g., small, medium or large) of the meal type to be / is being / has been eaten. In some implementations, the meal size can be displayed, for user input, prior to meal type. In implementations of the meal-type and size fixed-dose therapy dose calculator, each meal is associated with a pre-set dose size defined for the particular patient user and the particular selected, and this pre-set dose value would be displayed on a user interface of the device implementing the meal-type and size fixed-dose therapy dose calculator. In some implementations, if BG data (e.g., from a manual entry or a connected finger-stick meter or continuous glucose monitor) was available, the meal-type and size fixed-dose therapy dose calculator may also determine a correction dose for the patient user in this instance, and the sum of the correction dose and the pre-set dose size would be displayed as the complete dose to administer.

### Fixed-Dose Adjustment Titration Calculations Based On Meal Size and Content Estimation

In some embodiments, the disclosed methods, devices and systems include a fixed-dose titration calculator for automatically adjusting a total insulin for a patient user, where a first dose is recommended based on estimations of (i) a meal type or (ii) a meal type and size of the meal in conjunction with the patient's glucose level, and a corrective dose is recommended, when needed, based on the patient user's glucose level after the meal and first dose. The fixed-dose titration calculator is configured to determine a first, pre-set dose size of insulin for the patient to administer in association with a meal (and in some implementations, of a particular size) to be eaten, that is being eaten, or has recently been eaten, and to determine a second, adjustment dose size of insulin for the patient to administer within a time frame after the meal based on a measured glucose level of the patient user. The fixed-dose titration calculator can be implemented via a software application operable on a computing device, such as the companion device 5 (e.g., smartphone, tablet smartwatch, smartglasses, etc.), and/or a medical device, such as the medicament delivery device 10 (e.g., insulin pen). The software application can be configured as a standalone software application for implementing a dose adjustment via the fixed-dose titration or as an integrated feature of a software application directed to blood glucose level monitoring, insulin delivery, or other diabetes management application.

In implementations of the fixed-dose titration techniques for determining the first, fixed-dose based on meal type and/or meal size, the patient user may be instructed to input (e.g., select) the type of meal (e.g., breakfast, lunch, dinner, snack) to eat, being eaten, or has been eaten. In some implementations of the fixed-dose titration techniques for determining the first, fixed-dose based on meal type and/or meal size, the patient user may be instructed to input (e.g., select) the type of meal (e.g., breakfast, lunch, dinner, snack) to eat, being eaten, or has been eaten and the size of the meal (e.g., small, medium, large). In some optional implementations, for example, the patient user may be instructed, via a display produced by the dose calculator, to input (e.g., select) an estimated carb size of the selected meal, where the estimated carb size is based on the carbohydrate content of the food. For example, even if the user cannot count or estimate carbs accurately, they may be able to identify that some foods such as pasta and bread are high in carb, and other foods such as salad and yogurt are low, despite the physical size of the meal. Alternatively or additionally, for example, the fixed-dose titration technique can prompt for meal size (e.g., small meal, medium meal, large meal). The fixed-dose titration calculator may prompt the user to estimate for carb content, such as "low carb", "mid carb," or "high carb," and/or may prompt the user to estimate meal size prior to performing calculations to determine whether an insulin dose adjustment is needed, and if so, recommend a particular adjustment dose. The software application implementing the fixed-dose titration calculator can provide a user interface that may present training or reference material for foods that the user may be uncertain about. For example, this can provide the user with access to an online nutrition guide, or a general guideline or education about types of foods that are high or low in carbs.

The fixed-dose titration calculator can include a setup process to input information about the patient user and/or associated with the patient user's meal habits. For example, during setup of the user's settings in the software application, the patient user's physician may add individualized notes explaining the meal sizes, for example listing pizza as a high-carb example food. The physician and patient may go over some common foods and meal types and agree on how these should be characterized, storing these in the dose calculator software application for future reference by the patient user. Meals logged during use of the app could also be reported later to the physician, and as such, can enable the dose calculator software application to improve meal the fixed-dose adjustment calculations per estimated meal size and content. For example, as a corrective measure, the dose calculator app can list the foods consumed before particularly poor glycemic control events, along with the type of dose that were calculated, to be provided for the user's physician for review and discussion with the physician. This example corrective measure could apply to someone carb-counting as well - the food and the presumed amount of carbs could be reviewed by the physician and discussed if the estimate was inaccurate.

Notably, the setup process of the fixed-dose adjustment titration calculator app can be implemented for updating such input information throughout the course of the user's use of the app. For example, in addition to identifying particular foods that may have been mis-estimated, the app may also identify dose sizes that are not working well for the patient, which tend to lead to hyperglycemia or hypoglycemia (BG outside of some threshold of the target value or range). By comparing the average actual BG response to the desired BG response, the dose size may be adjusted accordingly. This could be done as an automatic adjustment by the software application of the fixed-dose titration dose calculator, with or without patient or physician confirmation. The fixed-dose titration dose calculator can present the determined dose size adjustment on the user interface as a recommendation to the patient or physician, allowing them to approve or make the change manually. Or it could be presented as a general trend of overcorrection or under-correction, and allow the physician to adjust the value an amount they deem appropriate.

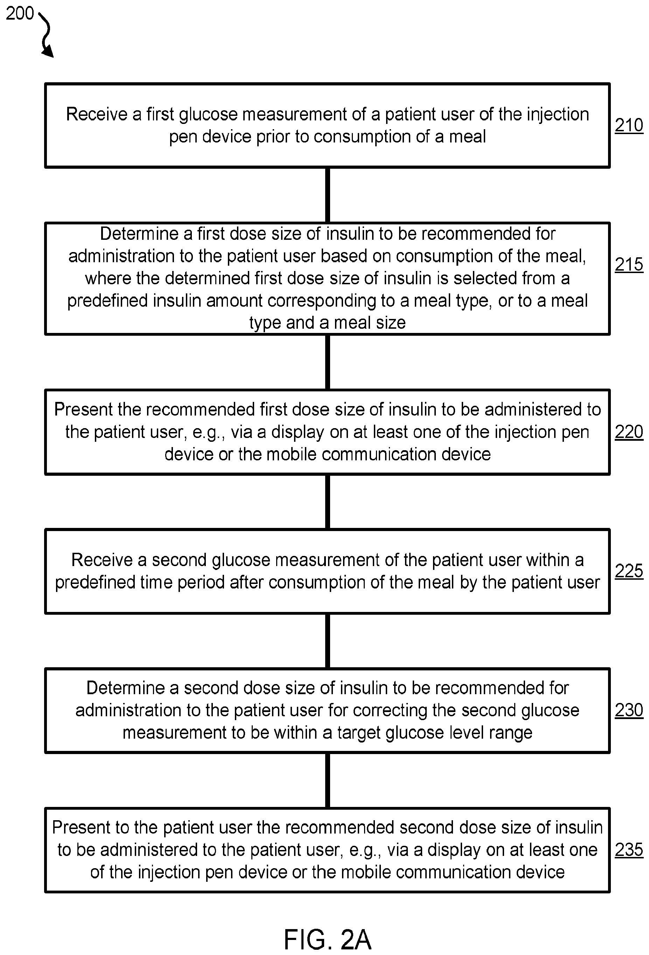

FIG. 2A shows a diagram of a method 200 for adjusting an insulin dose size by fixed-dose titration that can be implemented by the fixed-dose titration dose calculator operable on the system 100, e.g., on the pen device 10 and/or on the companion device 5, in wireless communication with each other. The method 200 includes a process 210 to receive a first glucose measurement of a patient user of the injection pen device prior to consumption of a meal. The method 200 includes a process 215 to determine a first dose size of insulin to be recommended for administration to the patient user based on consumption of the meal. In some implementations of the process 215, the fixed-dose titration dose calculator determines the first dose size of insulin by selecting the dose size from a predefined insulin amount that corresponds to a meal type and a meal size. The predefined insulin amount is estimated from an amount of carbohydrates estimated from the meal type and the meal size. Yet, in some implementations of the process 215, the fixed-dose titration dose calculator determines the first dose size of insulin by selecting the dose size from a predefined insulin amount that corresponds to a meal type. The method 200 includes a process 220 to present to the patient user the recommended first dose size of insulin to be administered to the patient user, e.g., e.g., via a display on at least one of the pen device 10 or the companion device 5. The method 200 includes a process 225 to receive a second glucose measurement of the patient user within a predefined time period after consumption of the meal by the patient user. The method 200 includes a process 230 to determine a second dose size of insulin to be recommended for administration to the patient user for correcting the second glucose measurement to be within a target glucose level range, e.g., including but not limited to: between 80 mg/dL to 130 mg/dL, between 70 mg/dL to 140 mg/dL, or a tighter range that includes 120 mg/dL. The method 200 includes a process 235 to present to the patient user the recommended second dose size of insulin to be administered to the patient user, e.g., via a display on at least one of the pen device 10 or the companion device 5.

In various implementations of the method 200, at the process 215, the meal size used in determining the first dose size of insulin to be recommended includes a small meal, a medium size meal, and a large size meal. In some implementations of the method 200, at the process 215, the first dose size of insulin is determined by selecting a predefined insulin amount based on the first glucose measurement and that corresponds to a meal type among the following meal types: breakfast, lunch, dinner, pre-breakfast snack, pre-lunch snack, pre-dinner snack, and post-dinner snack. Additionally, or alternatively, at the process 215, the first dose size of insulin is determined by selecting a predefined insulin amount based on the first glucose measurement and that corresponds to a meal type that accounts for a category of food in the meal. In an illustrative example, the category of food in the meal could be 1, 2, ... n slices of pizza, 1/2 plate or full plate of spaghetti, a 1/4 lb. hamburger, 3 pancakes with syrup, a BLT sandwich, etc. Furthermore, in some implementations of the method 200, the method 200 can further include, prior to the process 230 to determine the second dose size, a process to prompt the patient user, e.g., via the pen device 10 and/or the companion device 5, to provide a confirmation input that the meal was consumed by the patient user.

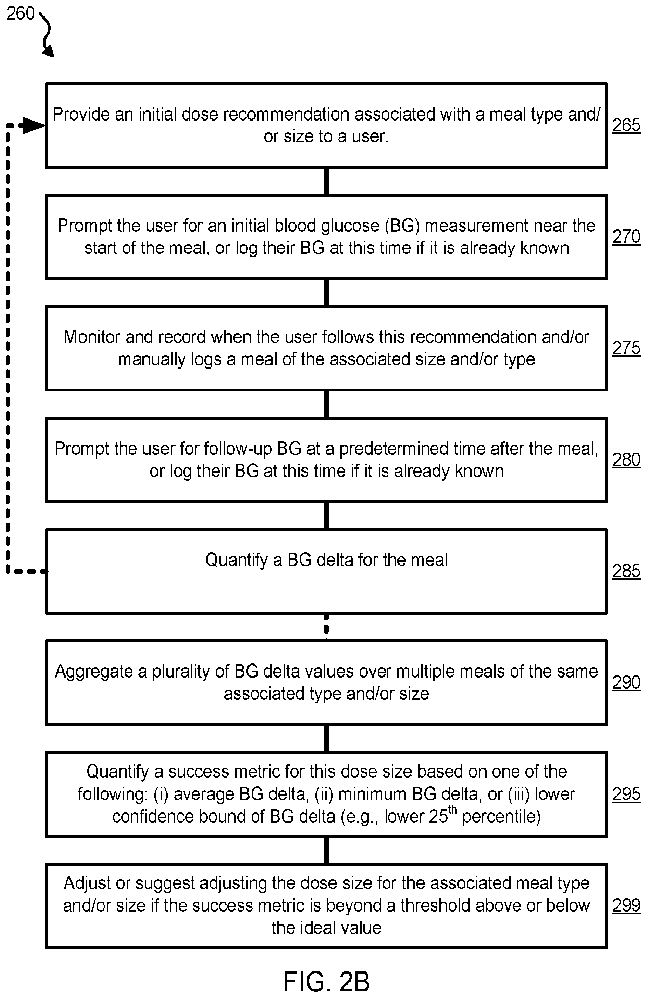

FIG. 2B shows a diagram of a method 260 for a calculating a dose size and/or dose adjustment size based on a fixed-dose titration technique involving meal dose size estimates for improving blood glucose control, which can be implemented by the fixed-dose titration dose calculator module, operable on the pen device 10 and/or companion device 5, or in some implementations in conjunction with the cloud. The method 260 includes a process 265 to provide an initial dose recommendation associated with a meal type and/or size to a patient user. In some implementations, for example, the recommendation may optionally be summed with a BG correction dose based on the user's current BG level. The method 260 can include a process 270 to prompt the user for an initial BG measurement near the start of the meal, or log their BG at this time if it is already known, e.g., such as via a connected CGM system. The method 260 can include a process 275 to monitor and record when the user follows this recommendation and/or manually logs a meal of the associated size and/or type. The method 260 can include a process 280 to prompt the user for follow-up BG at a predetermined time after the meal, or log includes their BG at this time if it is already known. The method 260 a process 285 to quantify a BG delta for the meal, i.e., a change in blood glucose level, where the BG delta includes a difference between follow-up BG and initial BG, subtracting the expected effect of the correction dose (correction dose divided by insulin-to-carb ratio) from the initial BG Described but not claimed is that the BG delta can include, e.g.,: a difference between follow-up BG and initial BG, or a difference between follow-up BG and the user's target BG.
The processes 265, 270, 275, 280 and 285 are repeated for various meals and corresponding BG measurements of the patient user. In this manner, the fixed-dose titration dose calculator module produces a plurality of BG delta values that are associated with multiple meals of substantially the same meal type and/or meal size where the processes 265, 270, 275, 280 and 285 are implemented.

As such the method 260 further includes a process 290 to aggregate the plurality of BG delta values over the multiple meals of the same associated meal type and/or meal size. The method 260 includes a process 295 to quantify a success metric for this dose size based on one of the following: (i) average BG delta, (ii) minimum BG delta, or (iii) lower confidence bound of BG delta (e.g., lower 25^{th} percentile). The method 260 includes a process 299 to adjust the dose size for the associated meal type and/or size if the success metric is beyond a threshold above or below the ideal value (e.g., of zero), which is implemented in the following way :
calculate a new associated dose by summing the initial associated dose with the success metric divided by the user's insulin-to-carb ratio. Described but not claimed are
the following ways: (i) suggest the user
increase the associated dose if the success metric is above a positive threshold, or suggest decreasing the associated dose if the success metric is below a negative threshold: (ii) increase the associated dose by a fixed increment if the success metric is above a positive threshold, or decrease the associated dose by a fixed increment if the success metric is below a negative threshold: .

**In** some implementations, a negative success metric, indicating the user often ends up low and is over-correcting, will reduce the recommended dose. A positive success metric will increase the recommended dose. In some implementations, adjustment may not be performed if the delta is below a threshold. In some implementations, adjustment may be limited to a maximum delta per adjustment period. In some implementations, the limits on adjustment delta may be greater for dose reductions than for dose increases, as this ens on the side of safety and avoiding overdose.

### Dose Calculator Setup

In various embodiments of the software application, the dose calculator module may include the option to switch between types of insulin dose calculation therapy, including a fixed-dose therapy (e.g., a set amount on insulin to take with any meal), a fixed-meal-dose therapy (e.g., a set amount of insulin to take for specific meals such as breakfast or lunch), a meal estimation therapy (e.g., a set amount of insulin to take for various sizes of meals, such as small breakfast or large lunch), and/or a carb-counting therapy (e.g., the user manually estimates the grams of carbohydrate in a meal). For example, depending on the option selected, the software application will display settings and recommendations for the selected therapy type as described herein.

During setup, carb-counting therapy requires an "insulin-to-carb ratio" parameter to be set that relates how many grams of carbohydrate a single unit of insulin can cover. All other meal dose methods do not require this parameter, because they simply prescribe a set amount of units without calculating based on grams of carbohydrate.

During setup, the software application may offer multiple options for meal type and size, such as breakfast, lunch, dinner, snack, dessert; and small, medium, large. However, not all values would need to be entered. By deactivating certain options or leaving them blank in the setup, the software would not present these options to the user for calculation and would simply display the populated options. Custom foods or meal types may also be prescribed. For instance, in addition to breakfast, lunch, and dinner, particular foods or meals that require special dosing could be listed explicitly, such as pizza, pasta, beer, etc. If fixed-dose therapy or meal estimation therapy was generally working well for a patient except for certain problematic meals, those could be included as separate options in the dose calculator with better-tailored dose recommendations for the user.

For example, the dose calculator setup can allow entry of (a) dose sizes for each general size and/or type of meal (e.g., lunch, small meal, big dinner); and (b) names of specific foods selected for the user (e.g., pizza) and associated dose sizes for these particular foods. Then when presenting a dose calculator interface, the software application offers recommendations for the general meal sizes and the specific foods entered for the user.

In another variation of the fixed-dose therapy, rather than prescribing the amount of units to take for various meals, the physician could prescribe an insulin-to-carb ratio and define carbohydrate values for the various meal types and sizes. Then at the time of use, the dose calculator would calculate appropriate doses for the patient based on the estimated carb amounts and insulin-to-carb ratio, though the user would not necessarily see the carb values, and would simply select a meal type and/or size.

### Simplifying Dose Calculator Setup

Some dose calculator therapies require setting many parameters - some of which may be tedious or time consuming, and others that may be difficult to calculate manually, especially for physicians who may be less-familiar with dose calculators or details of insulin therapy. To reduce this burden, techniques are disclosed to simplify, improve accuracy of, and automate the setting of dose calculator parameters.

### Streamlining Initial Setup

In the case of meal estimation therapy, there may be 12 or more dose sizes to set for the user. This may be tedious and prone to unintentional bias or miscalculation due to human error or judgement. Additionally, if these doses need to be updated to improve glycemic control for a patient, it may be difficult and imprecise to manually edit multiple entries.

One method for initial setup of a dose calculator in a software application would be to have the dose calculator obtain pre-set typical meal sizes (e.g., carb amounts) and apply an initial insulin-to-carb ratio for the user, where the initial insulin-to-carb ratio is configured based on input by a health care provider, including by a computing device of the health care provider. As an example, the insulin-to-carb ratio could be specified by the patient user's physician, or could be automatically calculated based on patient information (e.g., age, weight, etc.). For adjustment and refinement, specific meal carb amounts could be adjusted based on the user's diet, or the insulin-to-carb ratio could be adjusted to globally affect the doses recommended for all meal types and sizes.

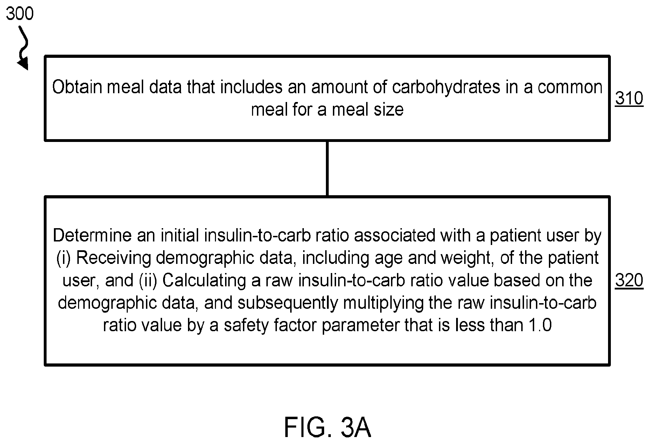

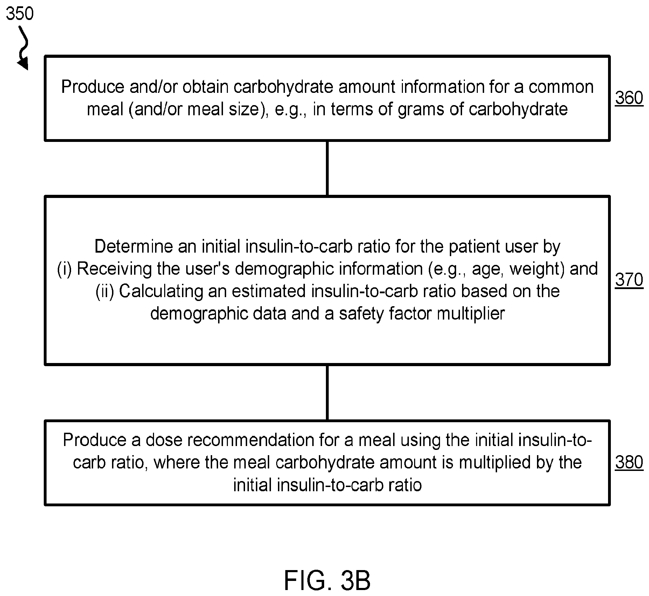

Yet, in some embodiments, a method for initial setup of a dose calculator in a software application includes providing carbohydrate information of common meals to the dose calculator, such as pre-set typical meal sizes (e.g., carb amounts), and generating an initial insulin-to-carb ratio for the user, where the initial insulin-to-carb ratio is configured to recommend doses that may err on a lower dose size amount as a safety feature to mitigate risks of overdosing by a new patient user. Example embodiments are shown in FIG. 3A and FIG. 3B.

FIG. 3A shows a diagram of a method 300 for setting parameters of an insulin dose calculator, which can be implemented by the dose calculator module, operable on the pen device 10 and/or companion device 5, or in some implementations in conjunction with the cloud. The method 300 includes a process 310 to obtain meal data that includes an amount of carbohydrates (e.g., in grams) in a common meal for a meal size. For example, the meal size can include a small meal, a medium size meal, and a large size meal. In various examples, the common meal can include pizza, pasta, hamburger, beer, salad, yogurt, French fries, or any other meal common to the country or specific locale of the patient user, which can be obtained through an existing database of meal information. The method 300 includes a process 320 to determine an initial insulin-to-carb ratio associated with a patient user of the injection pen device. The process 320 determines the initial insulin-to-carb ratio by first receiving demographic data, including age and weight information of the patient user, then by calculating a 'raw' insulin-to-carb ratio value that is based on the demographic data, and subsequently multiplying the 'raw' insulin-to-carb ratio value by a safety factor parameter that is less than 1.0. The determined initial insulin-to-carb ratio is thereby a reduced value than that of the 'raw' insulin-to-carb ratio due to the safety factor parameter. In some implementations of the process 320, the safety factor parameter includes an 80% (i.e., 0.8) multiplier. The method 300 includes a process 330 to provide the determined initial insulin-to-carb ratio to the dose calculator module such that the dose calculator produces a recommended dose of insulin by multiplying the amount of carbohydrates by the determined initial insulin-to-carb ratio. In some implementations of the method 300, the process 320 further includes (i) selecting a lower confidence bound of a confidence interval (e.g., where the lower confidence bound in a bottom 25^{th} percentile of a population based on the demographic data), and selecting the safety factor parameter accordingly (e.g., where the safety factor parameter is selected in a range of 60% to 80%).

FIG. 3B shows a diagram of a method 350 for setting parameters of an insulin dose dose calculator, which can be implemented by the dose calculator module, operable on the pen device 10 and/or companion device 5, or in some implementations in conjunction with the cloud. The method 350 can include a process 360 to produce and/or obtain carbohydrate amount information for common meals (and/or meal sizes), e.g., in terms of grams of carbohydrate. For example, these can be sourced from a central database, and may be based on demographic information of the patient user, such as age, weight, or special diets. The method 350 can include a process 370 to determine an initial insulin-to-carb ratio for the patient user, e.g., by (i) receiving demographic information, including age and weight information, on the patient user (e.g., which can be by prompting for manual entry via a display on the pen device 10 and/or companion device 5), and (ii) calculating an estimated insulin-to-carb ratio based on established demographic data, and a safety factor multiplier that is less than 1.0. The method 350 determines an initial insulin-to-carb ratio that mitigates risk for a new patient user, where generally a patient-specific insulin-to-carb ratio is only roughly estimated, it therefore desirable to err on the side of recommending smaller doses initially to avoid overdose. Implementing the method 350, this may be achieved by multiplying a raw insulin-to-carb ratio by a safety factor (e.g., 80%) to reduce the value of the insulin-to-carb ratio. Yet, in some embodiments of the method 350, the process 370 can include selecting a lower confidence value (e.g., 25^{th} percentile) of the population based on the demographic data. The method 350 can include a process 380 to multiply each of the meal carb amounts by the initial insulin-to-carb ratio to produce a dose recommendation for each of the meal sizes and/or types.

In some embodiments, a method for efficiently and promptly modifying settings of a dose calculator in a software application based on a physician recommendation or intervention is disclosed. For example, if a physician desires to adjust all dose recommendations quickly, a fixed amount could be added to all dose types, or a percentage offset could be applied. For example, the physician may quickly add 0.5 units to all dose recommendations, or increase all of them by 10%. This could also be applied to selected subsets (such as to all lunch dose sizes, or to all large meal types).

By entering a single dose recommendation for a particular meal size and type, all of the other dose recommendations may be automatically calculated based on typical ratios between values. For instance, if "medium lunch" was defined as 10.0 units, a small lunch may typically be 75% of this amount, and automatically populated as 7.5 units, a snack may typically be 40% of this amount and automatically populated as 4.0 units, and so on. The software may prompt for a particular type of meal to be set first, as it may be easier to correctly estimate and extrapolate from a "medium lunch" than a "small snack", but mathematically any value that is correctly set could produce estimates of all the others.

To complete this setup method, the system can contain predetermined factors of relative carb content for various meal sizes and/or types. For example, "medium lunch" may have a factor of 1.0, "large lunch" may have a factor of 1.5, and "small breakfast" may have a factor of 0.5. The system can prompt the user for the desired dose amount for one particular meal size and/or type. Divide this desired dose by its associated meal's relative carb content factor, and multiply that value by each of the other meal's factors to derive estimated dose sizes for each of the other meals.

### Initial Setup Based on Total Daily Insulin Dose (TDD)

There are common methods of estimating a user's ideal total daily dose (TDD) of insulin, and/or this value may be known for a patient based on previous insulin therapy. Yet, such conventional means for arriving at TDD are more or less achieved through trial and error. In contrast, the disclosed dose calculator module can be configured to advise a patient user of a total daily dose of insulin by establishing a target TDD first, and by accounting for the type of insulin the patient user administers.

In some embodiments, a method for setting parameters of an insulin dose calculator, which can be implemented by the dose calculator module, first includes establishing a target total daily dose (TDD) of insulin for the patient user. Second, TDD is split between long-acting and fast-acting insulin. Commonly this is desired to be a 50% / 50% split. Alternatively, a patient's desired total daily amount of fast-acting and long-acting insulin may be known from previous insulin therapy.

Long-acting insulin is typically taken once per day, so the patient would then specify when they would prefer to take the dose to set a reminder time. Alternatively, after using the system and logging long-acting doses, the software may recognize the typical time that a long-acting dose is taken and initiate a reminder if the dose is not seen within a threshold of this time. Some patients also take two long-acting doses per day, or a single dose every other day, and the same general method would still apply, adjusting dose sizes to maintain the desired daily amount of long acting insulin.

The desired daily amount of fast-acting insulin would then be divided across meals in a day, either based on typical ratios (e.g., 40% at breakfast, 50% at lunch, 60% for dinner) or allowing the user to distribute the daily insulin as desired across meals, based on the relative size of meals they consume.

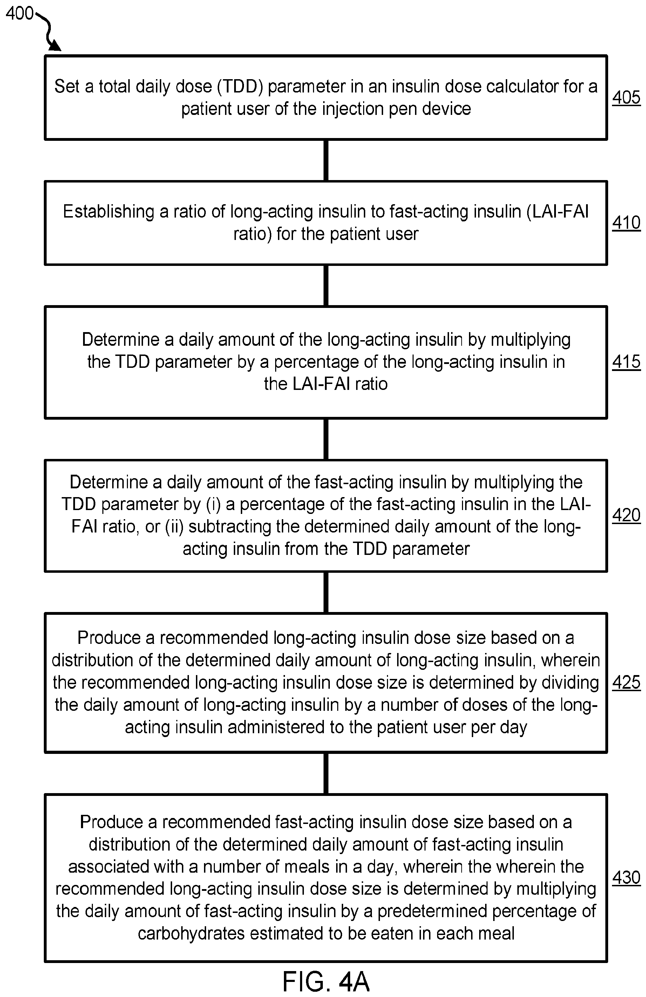

FIG. 4A shows a diagram of a method 400 for recommending an insulin dose based on a total daily insulin dose, which can be implemented by the dose calculator module, operable on the pen device 10 and/or companion device 5, or in some implementations in conjunction with the cloud. The method 400 includes a process 405 to set a total daily dose (TDD) parameter in an insulin dose calculator for a patient user of the injection pen device. The method 400 includes a process 410 to establish a ratio of long-acting insulin to fast-acting insulin (LAI-FAI ratio) for the patient user. The method 400 includes a process 415 to determine a daily amount of the long-acting insulin by multiplying the TDD parameter by a percentage of the long-acting insulin in the LAI-FAI ratio. The method 400 includes a process 420 to determine a daily amount of the fast-acting insulin by multiplying the TDD parameter by (i) a percentage of the fast-acting insulin in the LAI-FAI ratio, or (ii) subtracting the determined daily amount of the long-acting insulin from the TDD parameter. The method 400 includes a process 425 to produce a recommended long-acting insulin dose size based on a distribution of the determined daily amount of long-acting insulin, wherein the recommended long-acting insulin dose size is determined by dividing the daily amount of long-acting insulin by a number of doses of the long-acting insulin administered to the patient user per day. The method 400 includes a process 430 to produce a recommended fast-acting insulin dose size based on a distribution of the determined daily amount of fast-acting insulin associated with a number of meals in a day, wherein the wherein the recommended long-acting insulin dose size is determined by multiplying the daily amount of fast-acting insulin by a predetermined percentage of carbohydrates estimated to be eaten in each meal. In some implementations, for example, the predetermined percentage of carbohydrates estimated to be eaten in each meal can be derived from past insulin dose delivery size and time information over a past time period. In an illustrative example, if the patient user typically eats small-sized breakfast, a medium-sized lunch, and a large dinner, then the predetermined percentage of carbohydrates estimated to be eaten per meal can be 20% of your daily carbs are at breakfast, 30% are at lunch, and 50% are at dinner.

In some implementations of the process 405, for example, the TDD parameter can be set by receiving an input, via a user interface of at least one of the pen device 10 or the companion device 5, that includes a TDD value. In some implementations of the process 405, for example, the TDD parameter can be set by analyzing past insulin data to determine an average TDD value calculated over a predetermined time period, e.g., two days or more, three days or more (e.g., at least three consecutive days), one week, one month or other time period. In some implementations of the process 410, for example, the desired LAI-FAI ratio is established by recording an input, received via a user interface of at least one of the pen device 10 or the companion device 5, that includes the percentage of the long-acting insulin and the percentage of the fast-acting insulin. In some implementations of the process 410, for example, the desired LAI-FAI ratio is established by setting the LAI-FAI ratio as 50% long-acting insulin to 50% fast-acting insulin.

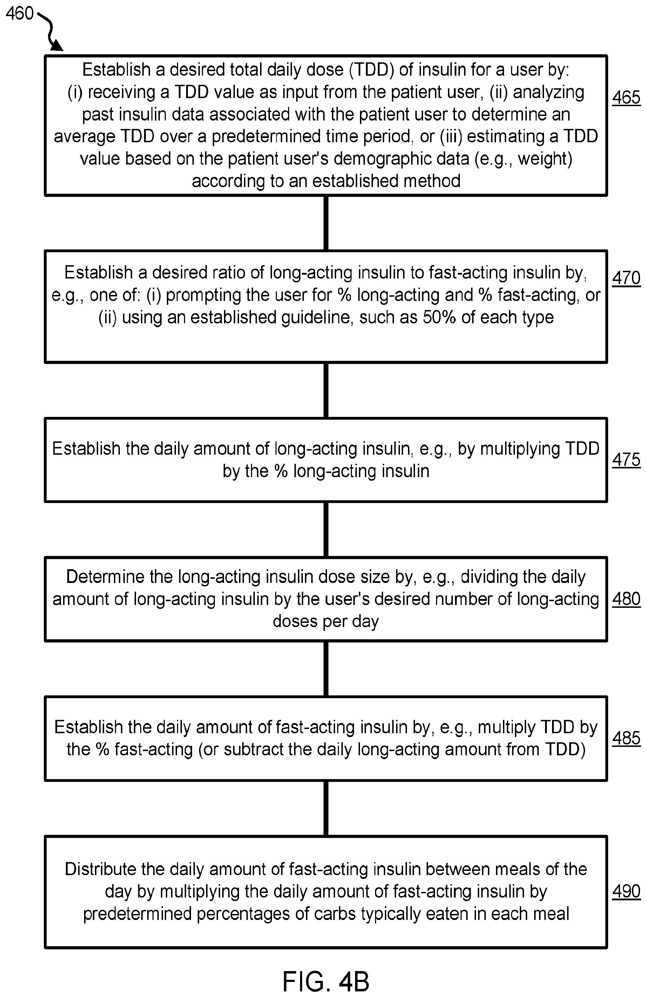

FIG. 4B shows a diagram of a method 460 for executing an initial setup of a dose calculator, e.g., such as a fixed-dose dose calculator, based on total daily dose (TDD), which can be implemented by the dose calculator module, operable on the pen device 10 and/or companion device 5, or in some implementations in conjunction with the cloud. The method 460 can include a process 465 to establish a desired TDD for a patient user, e.g., by one of the following: (i) prompting the user for TDD, (ii) reviewing past insulin data logs to determine the user's average TDD, or (iii) prompting for the patient's demographic data (e.g., body weight) that may be used to estimate a TDD based on established methods. The method 460 can include a process 470 to establish a desired ratio of long-acting to fast-acting insulin, e.g., by one of the following: (i) prompting the user for % long-acting and % fast-acting, or (ii) using an established guideline, such as 50% of each type. The method 460 can include a process 475 to multiply TDD by the % long-acting to establish the daily amount of long-acting insulin. The method 460 can include a process 480 to determine the long-acting dose size by dividing the daily amount of long-acting insulin by the user's desired number of long-acting doses per day (e.g., if they dose every-other-day, the value is divided by ½). The method 460 can include a process 485 to establish the daily amount of fast-acting insulin, e.g., by multiply TDD by the % fast-acting (or subtract the daily long-acting amount from TDD). The method 460 can include a process 490 to distribute the daily amount of fast-acting insulin between meals of the day, e.g., by multiplying the daily amount of fast-acting insulin by predetermined percentages of carbs typically eaten in each meal.

Alternatively, during setup, the user or physician may manually enter dose sizes for various meals, and the software would continuously calculate TDD (the sum of the daily doses entered thus far) and display the resulting TDD as dose entries are updated so that meal doses could be manually adjusted until the desired TDD is achieved.

### Meal Time Settings

A dose calculator can benefit from knowing typical meal times of the user for the purposes of providing suggested doses at the appropriate times, reminding or alerting the user when it is time to dose, and for alerting the user of possible missed meal doses.

The typical meal times may be set as time windows throughout the day. At the start of a meal time window the software may issue a notification, and display the recommended meal dose (or doses for multiple meal sizes) for that particular meal, for example lunch. At the end of the window if no meal has been logged or no dose has been taken, the software may remind the user of a possible missed dose, and may discontinue display of the meal suggestions since the window has passed.

The user-defined meal window may have a time threshold before the start and/or after the end in which the dose recommendations are still offered for that meal, accommodating a user who eats earlier or later than usual.

At any time the user may manually perform a dose calculation and/or log a meal. If a user logs a particular meal (e.g., lunch) before its preset time window, that meal would be considered eaten for the day, and the user will not be prompted for an entry again even when the normal time window begins.

Meals may also be set as a single setpoint, with a threshold applied to define a start and end time of a window. The threshold may be 30 minutes. For example, if lunchtime is set as 12:00pm, the app may issue a reminder and offer meal dose suggestions at 11:30am, and issue a missed-dose notification by 12:30 if no meal or dose has been logged. It is desirable to remind the user of a possible missed dose before discontinuing the display of meal suggestions, so that if the user responds to the missed dose alert they still have some time to view the suggested dose sizes and dose accordingly.

Alternatively, the app may always display options for the next meal. For example, once a breakfast meal or dose has been logged, it may immediately display lunch dose recommendations continuously until the user logs a meal or dose for lunch. Or the app may begin display of meal options at or near the beginning of the time window, but in the case of no meal or dose logged, may continue displaying them until the following window. For example if a lunch time window begins at 11:30am and dinner window begins at 5:30pm, it may display lunch dose calculations at 11:30, but if no lunch is eaten, it may continue offering the dose recommendations for lunch all afternoon until 5:30pm when the dinner window begins, and dinner dose recommendations are shown. In addition to convenience, immediately displaying calculations for the following meal may help to guide and educate the user as to what their next interaction should be - reminding them to return to the app at the next meal.

Weekends, days of the week, holidays, or other user-defined situations (for instance home vs. traveling) daily schedules may be set with times adjusted for the user's individual schedule.

### Simplified Setup and Adjustment of Meal Times

For simplified setup, meal times could begin as unset, or defaulted to typical meal times (such as 7am, 12pm, 6pm). Then after some time of using the app and logging meals, the app could automatically adjust the time windows to better anticipate meals and remind of possibly forgotten doses.

The app may set meal windows in one of the following ways, for example. The app may set meal windows from a set threshold (e.g., 30 minutes) before the earliest meal ever logged to a set threshold (e.g., 45 minutes) past the latest meal ever logged of the particular type (e.g., lunch). The app may set meal windows from a set threshold before the earlies meal to a set threshold after the latest meal of a type logged within a past time period, e.g., such as the past week or month. In some implementations, the app may determine the moving average time a particular meal is eaten by a user, and set the window from a set threshold before this moving average to a set threshold after.

A meal window does not necessarily have to correspond to the missed-dose reminder. For example, if a user typically eats lunch by 12:30pm but occasionally does not eat until 3:00pm, a missed dose reminder after 3pm would not be helpful on most days. In this case, the app may set the meal window extending past 3pm, meaning that lunch doses are suggested until 3pm or later, but set the missed dose reminder based on the average meal time, the median meal time, a rolling average meal time, or a user-defined reminder time. The reminder may occur a set time (e.g., 15 minutes) before or after this calculated or user-defined time as well. Reminding earlier gives the best chance to control BG by dosing closer to when food was likely actually eaten, but increases the occurrences of false alarms occurring before the user has actually eaten. Reminding later reduces false alarms, but means that if a user actually forgets a dose, it will be longer before they take the dose and will therefore have poorer glycemic control afterward.

### Defining or Refining Meal Times Based on Contextual Information

Meal times can also be inferred based on GPS (or a similar positioning system) location, activity monitors, or other contextual information. For example, if a user travels to a known restaurant, that may indicate that it is meal time. Or if a user sits at a desk for work (detected as a static location via GPS, or lack of activity by an activity monitor) and then GPS location changes or activity increases, that may indicate a lunch break. Typical meal locations may also be identified by GPS, as locations where the user has previously logged meals, taken meal doses, or where they have previously visited shortly after taking a meal dose or logging a meal. With precise indoor location information, this could include detecting the user moving from a desk to a breakroom, or from their living room to their kitchen.

This contextual information could provide meal recommendations, or more aggressively remind of a missed dose. For example, even if the user's typical reminder is at 1pm, if the app detects that they have been at a restaurant for some amount of time (e.g., 20 minutes) it may proactively remind the user to dose. For example, if they enter a restaurant at 10:50am, it may remind them at 11:10am to consider dosing for a meal, even though the usual time-based reminder is not set to occur until 1pm.

Additional 3^{rd} party contextual information may be used to identify meal times or missed doses as well. For example, a bank notification of a credit card charge from a fast-food restaurant or cafeteria (where one pays before eating) may indicate the user is about to eat, or a charge from a dining restaurant (where one pays after eating) may indicate that the user forgot to dose. These real-time notifications are already present in mobile banking apps and as text message or email alerts, so these types of notification could also be passed to the dose calculator software.

The user's location information could also be used to provide location-specific dose recommendations. For example, certain restaurants may be identified as higher or lower carb, and the dose calculator could suggest larger or smaller meal doses be selected. Or if the user visits a pub in the afternoon, the dose calculator may proactively offer doses to cover snacks, drinks, or appetizer foods, even though it was not a pre-set meal time for the user.

The user's history of doses, meal logs, activity, and/or location from before the dose calculator software is used can be used to improve the app's initial settings. For example, dosing history from a previous insulin device may be used to detect the user's typical meal dose sizes or total daily doses. Location history may infer the user's waking hours, meal times, and day-today schedules. Meal history (such as from a fitness or dieting app) may indicate typical meal sizes and times. In this way, even if the software has never been used by a patient before, it may still have historic data from other sources to learn from, or to base default recommended settings on. This data may be accessed directly from 3^{rd} party apps or cloud servers, or via consolidated repositories such as Healthkit or health apps.

### Multiple Meal Schedules

In addition to detecting a specific location, GPS (or similar) location information can be used to adjust a user's schedule. For example, location data may indicate that a user is at home for the day, at work, or on vacation, which then may modify the dose calculations and reminders. The user may explicitly set different schedules for these scenarios. Or, the learning functions of the app (such as automatic adjustment of dose sizes or meal times) may segregate the learning in one mode from another. For example, a user may eat lunch at 12:00pm when at home, but at 1:30pm when at school. By differentiating the locations of these meals, the dose calculator would set a lunch time window around 12:00pm when location information shows that the user is at home, and set a lunch time window around 1:30 when location information shows that the user is at school, or at least is not at home. Without this differentiation, the software would determine an average lunch time of 12:45pm, which would not be a useful setting at either location.

GPS or activity monitors could also be used to set meal times automatically based on when the user is sleeping. Both of these would detect sleep and travel patterns of someone who works a night shift, for example, and then may offset meal times according to their daily schedule. These users would also benefit from their correct meal schedule being selected based on the time they wake.

Time of breakfast can also be used to adjust time setpoints for the following meals in the day. For example, a user who works nights but sleeps at night on their days off may log breakfast at very different times on work days versus non-work days. An early breakfast may indicate that they will be awake during the day, having lunch around noon, and a late breakfast may indicate a workday where lunch would be in the evening. Time of breakfast could be used alone or along with other contextual information to determine which schedule to use for the rest of the day.

### Setting Parameters When Switching Between Therapy Methods

If a patient user switches between methods, the software may assist in calculating parameters for the new method based on past data and existing parameters. The software may algorithmically calculate recommended parameters for the new method, which may then be confirmed or modified as needed, which is simpler and more precise than manually setting all of the new parameters individually.

When switching from fixed dose to fixed meal dose, a typical offset may be applied to the fixed-dose size to estimate breakfast, lunch, and dinner doses. For example, breakfast may be set to 80% of the previous fixed dose, lunch set to 100% of the previous fixed dose, and dinner set to 120% of the previous fixed dose. This maintains the same total daily dose of insulin, but spreads it more appropriately across meals. These percentages could be recommended based on typical values, or could be set or adjusted by the user for their personal eating habits.

Switching from fixed meal dose to meal size estimation, the doses for each meal may have an offset applied to calculate "small", "medium", and "large" variants. For example, small may be 75% of the previous fixed meal dose, medium may be 100%, and large may be 125%. For example, if the user's lunch dose was set to 10 units, the software may automatically propose 7.5u small lunch, 10.0u medium lunch, and 12.5u large lunch.

Switching from any of the fixed or meal estimation dose methods to carb-counting requires an insulin-to-carb ratio parameter to be set. If the user or physician estimates the typical carb content for any of the already-set meal doses, or if a common carb content value is proposed by the app, this can be used to calculate the user's effective insulin-to-carb ratio. For example, if the user's lunch dose is set to 10u, and a typical lunch is estimated at 30 grams of carbs, the software may automatically calculate and propose an insulin-to-carb ratio for the user of 3 grams per unit to be used in the dose calculator in carb-counting mode.

In the case where a user has established an insulin-to-carb ratio but desires to transition to a simpler method such as meal size estimation, the insulin-to-carb ratio can be used to calculate and propose doses for various meal sizes. The software may use previous data for common meal sizes and carb content, it may reference past meals logged by the user, or the user or physician may estimate typical grams of carbs in the patient's meals. Then based on that, dose sizes can be calculated. For example, with an insulin-to-carb ratio of 5 grams/unit and a presumed lunch meal size of 30 grams of carb, the software would propose a 6 unit dose size for lunch meal.

### Correcting Low Blood Glucose

When an insulin-to-carb ratio is known and a user is below their target BG, this can be used to calculate the grams of carbs the user should consume to return to target. However, for fixed-dose or meal estimation therapy, the insulin-to-carb ratio may not be defined, and the user may not know how to correlate a carb recommendation to which food to eat. In this case, an alternate solution is needed.

The user may be informed to simply eat or drink without a quantity. As long as the user consumes enough carbs, this is safe, even if they have too much and reach a high BG level, at least they avoid hypoglycemia which is more immediately dangerous.

The software may differentiate BG that is slightly below target from BG that is dangerously low, and offer different alerts accordingly. Slightly low (e.g., 75 mg/dL) the software may indicate that the user should eat and monitor their BG, whereas when dangerously low (e.g., 50 mg/dL) the software may indicate an emergency and insist that the user eat carbs immediately and call for help. For users who do not have continuous glucose monitoring, this may also set a timer to remind the user to check BG again after some time (e.g., 30 minutes) has passed.

A dose calculator will typically be programmed with the user's "insulin sensitivity factor", or, the expected change in BG per unit of insulin, and also be programmed with the user's target BG value (or use a safe default, such as 120 mg/dL). Based on this information, when a patient is low, the app can back-calculate how many units of insulin correspond to the difference between actual and target BG. Then, knowing this number of units, it can correlate that to one of the pre-set meal doses, and recommend that size meal to the user to correct their BG.

As an illustration, if a user's target BG is 120 mg/dL and their current BG is 80 mg/dL, this is a delta of 40 mg/dL. If their insulin sensitivity factor is 10 mg/dL per unit of insulin, then the 40 mg/dL delta correlates to 4 units of insulin. And if the physician has set the app to recommend 4 units of insulin for a snack, the software would recommend eating 1 snack to correct the BG to target.

### Method for Calculating Correction Meal Without Carb Ratio

In some implementations, the dose calculator module may also recommend a meal, or a certain size snack or meal. It may also indicate some fractional amount of a meal, such as ½ a small lunch, or 2 snacks.

Identification of BG outside of the target range may be done locally on the user's device, or remotely based on glucose data in the cloud, and used to push a notification to the user to take corrective action.

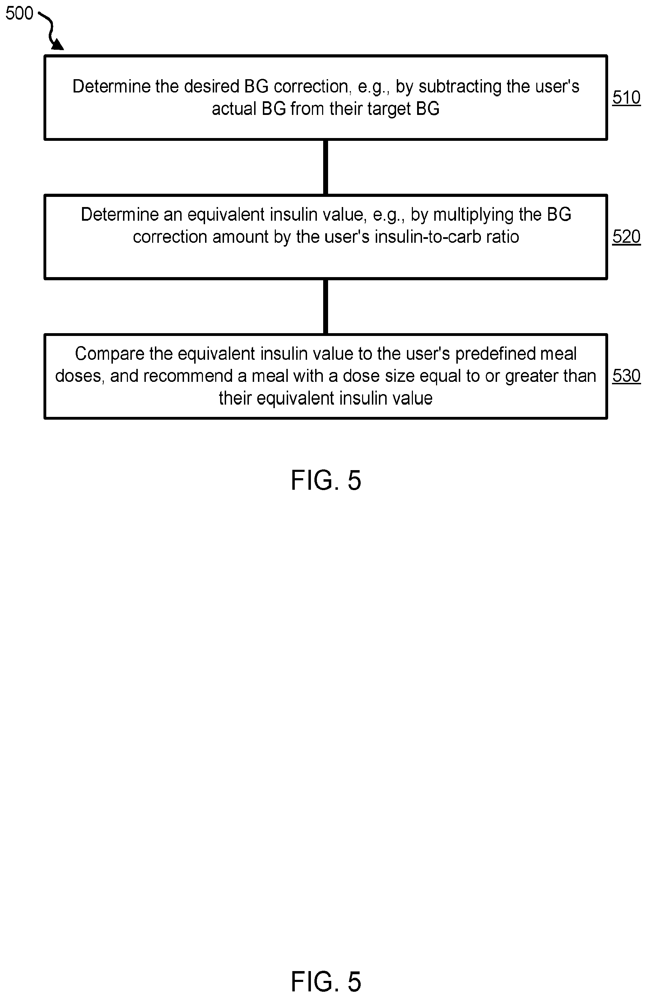

FIG. 5 shows a diagram of a method 500 for calculating a correction meal recommendation, e.g., without a defined insulin-to-carb ratio. The method 500 can be implemented by the dose calculator module, operable on the pen device 10 and/or companion device 5, or in some implementations in conjunction with the cloud. The method 500 can include a process 510 to subtract the user's actual BG from their target BG to determine the desired BG correction. For example, if this delta is below a predetermined threshold, no recommendation may be given to the user. The method 500 can include a process 520 to multiply the BG correction amount by the user's insulin-to-carb ratio to determine an equivalent insulin value (the amount of insulin that would normally cause a change in BG equal to their current amount below target). The method 500 can include a process 530 to compare the equivalent insulin value to the user's predefined meal doses, and recommend a meal with a dose size equal to or greater than their equivalent insulin value. For example, meal suggestions may be limited to the meal typically eaten at the current time of day - for example, displaying lunch recommendations at the user's defined lunch time. For example, meal suggestions may always default to "snack" sizes. If it is not a meal time for the user or if they have already recently eaten a meal, for example, meal suggestions may be limited to "snack" sizes. In some implementations, if there is not a meal dose to suggest that is roughly equivalent to the equivalent insulin value, fractional or multiple values may be recommended to result in the appropriate equivalent insulin dose value. For example, the system can determine the number of snacks to eat by dividing the equivalent insulin value by the dose size for a snack (e.g., if the equivalent insulin value is 8u and a snack has a dose size of 4u, 8/4 = 2 so the system would recommend 2 snacks).

The physician may customize recommendations for the user if they are below target BG. For example, considering their patient's physiology and typical foods, they may specify an apple at 20 mg/dL below target, a glass of juice at 40 mg/dL below target, and glucose tablets 60 mg/dL below target. These customized foods could be listed at different glucose levels, or at different deltas from target. Then when the patient is below target, the software would display the appropriate correction food based on these settings. This could also include personalized messaging such as, "check your BG again in an hour" or "call your mom immediately" to be displayed alongside the food recommendation in the software.

The software may also recommend exercise to correct high or low BG in lieu of or in addition to food or insulin. The physician may set a BG threshold for the app to recommend brief exercise, or may define the expected change in BG for this patient, and the app would then calculate when it would be appropriate to help return the patient to target BG. Additionally, if the user planned to exercise, they could manually enter this into the dose calculator so it could adjust the dose or meal recommendations accordingly.

### Touchless Bolus Calculator

### Overview

"Touchless" indicates that the user does not need to manually initiate a dose calculation (e.g., by touching the user interface), because recommendations are automatically passively displayed. And upon dosing with a smart insulin pen or other insulin delivery device that automatically logs doses to the dose calculator, the calculator updates or dismisses the recommendations and logs the dose and corresponding meal or calculation information, again without user interaction. The user may choose to interact with the interface to refine the recommendations or log additional data, but it is not required.

The touchless bolus calculator is designed to provide the patient with the best dose recommendations possible based on available data, and prompt for optional additional input when it is useful or there is high uncertainty. These recommendations are provided proactively based on context, without the user needing to specifically run a calculation or enter information. They may be displayed in an app home screen, a lock screen widget, on a smart watch, or similar.

The touchless dose calculator bases calculations on the user's recent meals, recent doses, current and recent blood glucose levels and trend, the time of day, and the settings and scheduling described above (which may be further refined based on activity monitors, GPS, 3^{rd} party health data, and other data sources as described). This contextual information, along with the calculation and dosing parameters set for the particular user, is used to generate real-time recommendations.

Dose recommendations are displayed automatically without user input, and after a meal dose is taken, the recommendation is dismissed automatically without user input.

### Dose Recommendation Scenarios

If BG is above or below target and it is not a meal time, the calculator will display the calculated dose (bolus) or food required to return to target. This could be pushed to the user via a notification, or it may be passively displayed continuously, allowing the user to decide when a correction is warranted.

At meal time (determined based on settings described above), the touchless bolus calculator will display the appropriate dose for the meal (for fixed meal dose therapy), or will display several options based on meal size (for meal size estimation therapy). Each recommendation will correlate to the pre-set or calculated insulin dose required to cover the meal.

In addition, if the user has entered a recent BG or has automatic BG data available (such as from a continuous glucose monitor) this will be accounted for in the calculation. As with a traditional dose calculator, the insulin required to correct BG to target will be summed with the meal dose to provide a total "meal + correction" dose. If BG is below target, the insulin required is effectively a negative value and will therefore reduce the overall recommendation when summed together.

If no recent BG data is available, the app can still display the meal recommendations so if a user is comfortable with their current BG level and does not desire a correction, they can take the appropriate meal dose without having to enter information. However, the app may also prompt the user to enter a BG so that it can be accounted for in the calculation, improving glycemic control. This may be a notification, or a pop-up message upon the user's selection of a meal size, encouraging them to enter a BG value to improve the recommendation.

If BG is below target, some meal options may require no insulin and will be shown with 0 units required. If BG is further below target, some meal options might not even be enough to return BG to target, so they may be accompanied by a warning message, or may be deselected or hidden, indicating that the user should eat a larger meal to correct their BG.

If a user has eaten recently (such that food is still being digested) as indicated by a logged meal or meal dose, then typically a BG correction dose is not recommended immediately afterward (e.g., for 2 hours). In these cases, the app would not prompt the user for BG since BG correction is not recommended anyway. If BG was already available to the app, by default it would not use this in the calculation, and would either disallow BG correction or would require user confirmation to include it.

An alternate approach to blocking BG correction for some time after a meal is to attempt to model the effect the carbs are likely to have on BG over the time period. Immediately upon eating, BG may not have responded at all and the full BG effect of the meal is still expected to occur. Some time later a portion of the food has been digested, related to the time elapsed since eating and the food's dynamic glycemic response, so a portion of the food's overall effect on BG has already occurred, and a portion is still expected. This continues until the food is completely digested and it will not affect BG any further. If this is modeled and accounted for, it is not necessary to ignore current BG and insulin on board, and this can all be summed into the final dose recommendation, including BG correction.

### Maximum Dose Limit

Commonly a patient's dose calculator will be programmed with a "maximum dose" that limits the size of any single dose, and limits the aggregate of doses within some time period such as 2 hours, or at least warns if the cumulative amount exceeds this maximum.

In the touchless dose calculator, this would be implemented by limiting the maximum dose sizes displayed in the automatic recommendations. Even if the patient's BG was extremely high and they were eating a large meal, the dose recommended would be limited to the preset maximum dose for safety. This may set a timer to remind the patient to check their BG sometime later (e.g., 2 hours) to confirm their adjusted BG level and further adjust if needed.

If the patient's recent doses plus the current recommendation exceed the maximum, the current recommendation may be shown with a warning that the cumulative doses exceed their set maximum.

If the patient's recent doses already exceed the maximum, the dose calculator may not offer automatic dose recommendations, and only allow manual calculations if the user initiates this interaction.

When a patient's dose or combination of doses exceed the set maximum dose, the app may show the user a warning, and may send a message to caregivers via text message or notification in a remote monitoring application.

### Prime Differentiation & Dismissing Recommendations

Typically users will prime a pen (dispense onto the ground to clear air from the pen) and then dose. It is desirable to dismiss the recommendation after the dose has been taken, but not after priming, as the user has not taken the dose yet and may still need to refer to the recommended size or confirm that they haven't taken it yet if they get distracted. Several solutions may exist for this.

The smart pen may be equipped with an explicit dose/prime sensor, such as a sensor that detects skin contact, orientation, body proximity, or a manual user-set toggle to indicate dose versus prime. In this case, the pen indicates that the prime is not the therapeutic dose, and the recommendation is not dismissed until the therapeutic dose is given.

Often the prime will be small and the recommended doses may be significantly larger. By determining that the initial priming was smaller than the lowest recommendation by a set threshold, the system determines that this was not the therapeutic dose. For example, if 4u is the smallest recommendation and the user dispenses 2u, the system determines that it was priming.

Typically, the pen is primed immediately before a dose, with only several seconds or minutes between, so if the smart pen is actuated and it is ambiguous whether it was the dose or a prime, the system may persist displaying the recommendation for a set amount of time (e.g., 30 seconds) after the last dose was received. This way if the user still needs to dose, the recommendation is still visible.

After the system determines that a dose was taken and dismisses the recommendation, it may still be displayed in an altered way, or with an icon or other notification indicating that it was a past indication. This way the user is not prompted to dose again they can still reference the applicable value.

The user's past behavior of priming can be used to better inform the software. While users are always instructed to prime before dosing, often the decision of whether to prime or not is a personal one and behavior is consistent. So, a user who has primed before doses in the past can be expected to prime again, so the first actuation of the smart pen is likely a prime. A user who rarely or never primes before doses can be expected not to prime, so the first actuation of the smart pen is likely a dose. For example, by referencing past data where all pen actuations are known (as opposed to real-time data where there may or may not be more actuations upcoming) the system may determine the frequency at which a user primes the pen before taking a therapeutic dose. If the frequency of priming is above a set threshold, the system may designate the user as one who typically primes their pen. In this case, the first pen actuation within a time window may be designated a prime, even if it would otherwise be ambiguous based on size or other factors.

### Implicit Logging of Assumed Meals and Calculations

For insulin dose calculators, when a patient user manually enters data into the dose calculator (as opposed to tapping an automatically-displayed suggestion by the dose calculator), this manually-entered dose can be saved for future reference by the dose calculator, and/or included among reported metrics showing utilization and compliance of the patient user with their insulin treatment regimen. However, when a dose is taken based on an automatically-displayed dose, and the patient user does not interact with the dose calculator, no dose would be logged (and the recommended dose calculation would be validated), even if the user did in fact reference it for their dose. So, in the case of a touchless dose calculator, when a user administers to him/herself a dose using the pen device 10 that matches or is similar to a recommended insulin dose by the touchless dose calculator, then the touchless dose calculator presumes that the patient user followed the recommendation, and thereby, the recommended dose calculation is logged and it is recorded that the patient user complied with the recommendation. Notably, this saved dose calculation may include the parameters used for calculation, and the displayed recommendation. It may display all displayed recommendations, or just the one presumed to have been followed by the user. In this regard, the touchless dose calculator is able to validate recommended doses without direct interaction by the patient user with the dose calculator.

In addition to not requiring user input to display dose calculations, the touchless dose calculator does not require the patient user input to log meal doses. If dose recommendations are displayed at a meal time, and the patient user takes one of the recommended doses, the software application will assume that the dose was for a meal, and estimate the meal size based on the corresponding recommended dose. For example, at 12: 10pm if the software app associated with administration of insulin by the pen device 10 displays 2 units for small lunch, 4 units for a medium lunch, and 6 units for a large lunch, and the user doses 4 units, then the app will infer that the user ate a medium lunch and dosed accordingly. It will also record that the user correctly followed the dose calculator for reporting compliance metrics to the physician.

The paring of doses to meals, or the logging of presumed meals can be done locally on the user's device, or after the fact on a separate computer or cloud server.

Presumed meal logging, and the pairing of doses to meals, may be used to dismiss meal dose recommendations, to report on dose calculator compliance (e.g., how often the calculator was followed) and therapy compliance (e.g., how often the user remembered to dose for meals), and to notify other caregivers in real-time via remote monitoring.

The user may also manually adjust past meal logs and presumed meals. Reviewing past logs in the software, they may adjust meal sizes, add missing meals, or indicate that a dose was or was not taken for a meal. This could be done to improve accuracy of reports, or to correct the real-time dose recommendations. (Such as if a correction dose was taken in the morning and the software assumed it had been for lunch, and then had dismissed the lunch dose recommendations. Correcting the history would then cause the lunch recommendations to be displayed again, which the user needs for when they actually have lunch, for example.

In some embodiments, a method for implicitly logging meals includes displaying one or more dose recommendations to the user; monitoring an insulin delivery system to detect when a dose equal or nearly equal to a recommendation is taken; and logging a meal of the type and/or size correlated with the recommendation at the time of the dose.

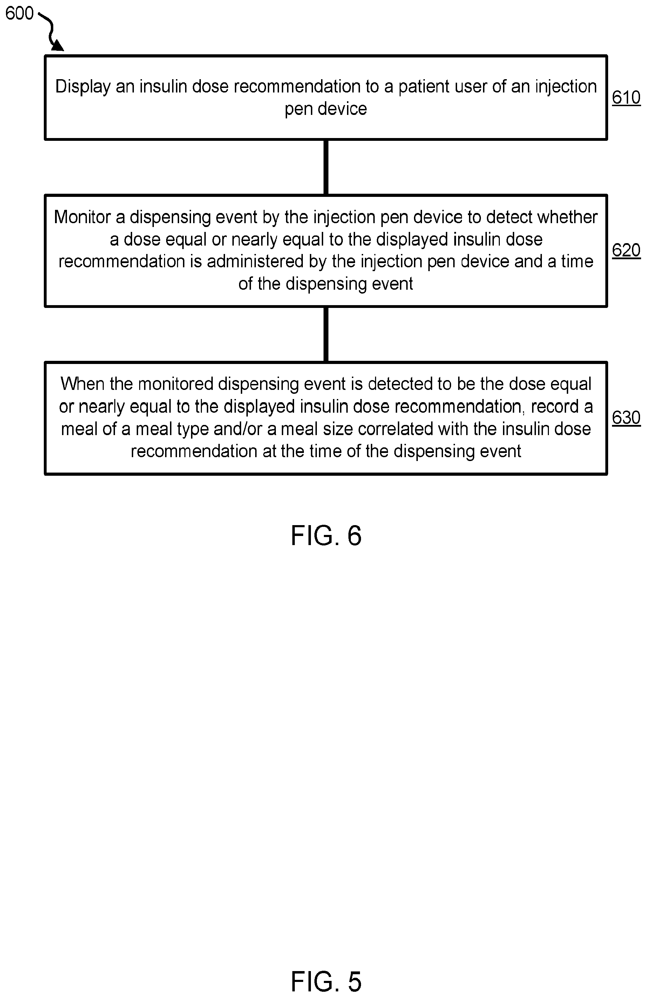

FIG. 6 shows a diagram of a method 600 for autonomous insulin dose recording without user interaction for an insulin dose calculator, which can be implemented by the dose calculator module, operable on the pen device 10 and/or companion device 5, or in some implementations in conjunction with the cloud. The method 600 includes a process 610 to display, via a display on at least one of the pen device 10 or the companion device 5, an insulin dose recommendation to a patient user of the pen device 10. The method 600 includes a process 620 to monitor a dispensing event by the pen device 10 to detect whether a dose equal or nearly equal to the displayed insulin dose recommendation is administered by the pen device 10 and a time of the dispensing event (when such the equal dose or nearly equal dose is dispensed). In some implementations of the process 620, the dose nearly equal to the displayed insulin dose recommendation is at least 90% of an amount of insulin as the insulin dose recommendation. The method 600 includes a process 630 to record, at the dose calculator module, meal information including one or both of a meal type and a meal size correlated with the insulin dose recommendation at the time of the dispensing event, where the process 630 is implemented when the monitored dispensing event is detected to be the dose equal or nearly equal to the displayed insulin dose recommendation at the process 620.

In some implementations of the method 600, the meal type is selected from a group consisting of breakfast, lunch, dinner, pre-breakfast snack, pre-lunch snack, pre-dinner snack, and post-dinner snack. In some implementations of the method 600, the meal size is selected from a group consisting of a small meal, a medium size meal, and a large size meal. In some implementations of the method 600, the meal type includes a food category (e.g., such as pizza, pasta, beverage type, hamburger, pancakes, etc.).

### Doses Not Matching a Recommendation

If a dose is taken when automatic calculations are displayed but the amount does not exactly match a recommendation, there are several options for handling this:

The software may round up, round down, or round to the nearest dose recommendation and log that. It may also log this nearest recommendation and log that the user overrode the recommendation by some delta.

The software may explicitly prompt the user for confirmation of whether the dose was for a meal, and if so, what size was estimated.

It may simply be logged as a non-meal dose. This may be done for any dose that does not exactly match a recommendation, or for doses some amount beyond the range of minimum to maximum recommendations.

The software may interpolate between recommendations to determine the meal size the user had assumed. This could be on a qualitative scale like "Medium-Small" or on a quantitative scale of carbohydrates, for example interpolating between recommendations of 20 grams and 40 grams to presume that the user had estimated 30 grams, based on their administered dose size.

### Doses Taken Outside of a Meal Time Window

Doses taken outside of a defined meal window could be presumed to be for the next meal if within some time threshold of the usual set meal time. Or upon dosing the user could be prompted, for example, "Did you eat a meal?" or "was this a meal dose?" This would ensure that appropriate reminders and recommendations are displayed to the user, and that the correct metrics of missed meal doses are reported, even if the meal was eaten outside of the usual time.

When meal doses are observed outside the pre-set meal time windows, this could be used to adjust meal time settings, or to suggest that the user adjust the settings.

### Touchless Dose Calculator for Carb-Counting Therapy

A patient on carb-counting therapy may set common meal sizes in terms of grams of carbohydrate, and near meal time the displayed dose recommendations would be based on these common meal sizes.

The user could mentally interpolate between recommendations if one did not exactly match their estimate of the meal. For example, if a user estimated 30 grams of carbs for their meal, and was presented the calculations: 4 units for a 20 gram meal and 8 units for a 40 gram meal, they could easily interpolate 6 units for their 30 gram meal. In this way a touchless dose calculator can be used for carb-counting therapy, even without the need to enter the exact amount of carbs.

The interface could also allow refinement of the recommendation, showing example doses as described above, but allowing the user to adjust these recommendations as well. Tapping a dose recommendation may present an interface for fine-tuning this value, such as a slider or up/down arrows to adjust grams of carb, or displaying a new set of recommendations clustered around this value. For example the software may display doses for 20, 40, and 60 grams, but upon tapping 40 grams it may then display doses for 30, 40, and 50 grams.

### Prompts to Check BG

In addition to meal times, other important times of day may warrant recommendations.

For example, before bedtime, the touchless dose calculator may produce a prompt for the user to check their present BG level; or, if the patient user uses a continuous glucose monitor (CGM) device, the touchless dose calculator may produce a command to obtain the present BG level from the CGM device. When the BG data is available, the touchless dose calculator may recommend a correction dose (or, alternatively, eat a meal) before the patient user's typical bedtime, or the touchless dose calculator may assure the user that their BG is acceptable and no further action is expected to be needed for the night.

In such implementations, bedtime can be determined based on a user-defined setting in the touchless dose calculator, based on current or past data from activity monitors and/or GPS location data, and/or from 3^{rd} party apps or built-in mobile OS functions, such as bedtime reminders.

The end of the day, when no further action is needed, is also a time when statistics or a summary of the day can be presented. This may include metrics of compliance or missed doses, glycemic control, high and low BG of the day, comparisons of this day's glycemic control to past days or historic averages and trends.

The summary may also include encouraging messaging about successfully managing diabetes for the day, setting a new record for compliance (e.g., days in a row with no missed doses) or control (e.g., best high & low BG or most time in range), or persisting through difficulties encountered (e.g., hypoglycemia or poor BG control), based on the patient's actual experience of that day.

These results could also be used to prompt revisions to therapy or setting an appointment to meet with a physician.

The user may enter their own diary comments or notes about good or bad experiences, to be stored in the database. These could be reviewed by the physician and help fill in gaps in the patient's memory about what happened on notable days, or to highlight issues to discuss at an appointment.

Even if a user skips a meal, and logs in the software that they will not be eating, the software may still prompt for BG, or if BG is known, may recommend a correction dose near the user's typical mealtime.

### Dose Calculator Interfaces

### Proactive Automatic Recommendations

A dose calculator may be displayed in the home screen of a mobile app, on a smart watch display, on a smart phone lock screen or home screen widget, or similar. The touchless recommendations and logged doses, meals, and calculations may be displayed alongside or on top of other controls, settings, and status displays. The options and recommendations appear when they are useful (such as during a meal window) and are dismissed, minimized, or hidden when they are not likely to be useful.

When the next scheduled activity (meal time, check BG reminder, etc.) is at least some minimum amount of time (e.g., 1 hour) in the future and BG is acceptable, the interface may communicate to the user that there is nothing to do for the next time period. This may help calm the user and reduce stress, focusing on the break in activities rather than focusing on future needs.

### Schedule Checklist Interface

In one embodiment of an application display (e.g., mobile app on a smart phone) the user is presented with a checklist for their day. In the morning, this may consist of a basal dose, meal doses, and times to check BG (e.g., 2 hours after each dose, or before bedtime). The checklist is blank when the user wakes up, and as the items are completed they are checked off.

If the user uses CGM instead of taking fingersticks, rather than prompting to check BG on at scheduled times, the app may automatically confirm whether BG is in range at these set times, and either confirm good or recommend a correction.

Throughout the day, additional unplanned line items may be added, such as correction doses, snacks, high or low BG alerts, cartridge replacements, temperature or battery alerts for the insulin device, and any follow-up fingersticks that are scheduled such as after a BG excursion.

Additional planned line items related to diabetes management could include other medications to take, replacing a CGM sensor or pump infusion site, changing a pen needle, replacing expiring insulin, scheduling or attending a physician appointment, generating a report and sending it to a physician, exercise reminders, activity monitor goals (e.g., 5000 steps for the day), and drinking water.

When the time for one of the planned line items approaches, it may become active and highlighted, and its display may change from a task item to an interactive element where the user may log an entry, view a recommendation, or edit content. For example, an item may initially be labeled "Lunch", but near lunch time it displays recommended doses for various meal sizes, and after being completed it displays the dose and meal information and allows it to be edited in context.

If the time passes and one of the checklist items has not been completed (e.g., forgetting to take a lunch dose) the item may be further highlighted and brought to the user's attention in a notification directing them to complete the missed item.

If an unexpected need arises, such as a high BG alert, the checklist would receive a new line item at the current time prompting for a correction dose, and recommending the amount to take. Once the dose is taken, the line would be checked as complete.

Optional tasks, such as corrections for BG just slightly out of target range, or additional fingersticks to be taken at the patient's discretion, may temporarily be displayed at the current time. If the user elects to take a very small correction dose or log a small snack to adjust BG then it is logged as a checked-off task, but if not then the temporary item eventually expires and is not maintained on the checklist.

The checklist serves as a history log and an educational tool, helping users understand how and when to interact with the dose calculator to manage their diabetes.

Completed checklists from past days may be viewed as a history log. The patient may highlight entries or add notes for future reference or review with a physician. These notes and entries may be included in exported physician summary reports.

When the next item in a checklist is at least some minimum amount of time (e.g., 1 hour) in the future and BG is acceptable, the future checklist items may be grayed-out or de-emphasized visually, and a line item at the current time may tell the user that there is nothing to do for the next time period. This may help reduce stress, focusing on the break in activities rather than focusing on the remaining checklist items.

The checklist interface may be implemented as follows. The system can determine the user's daily dosing schedule including one or more of: (i) long-acting insulin doses; (ii) meals; (iii) BG checks following meals or doses; and (iv) system-specific items such as replacing an expiring device (e.g., sensor, infusion site, pen, pump, or replaceable battery) or nearly-empty or expiring insulin container. The system can populate the predetermined schedule items in chronological order in a list or checklist format. The system can show applicable dose recommendations for a current task based on meal estimation and/or the user's current BG. The system can designate list items as complete when one of the following occurs: (i) the user manually selects an item as completed, (ii) a dose is administered corresponding to a current recommendation, (iii) or the system detects replacement of a device (e.g., a new smart insulin pen is connected) or an insulin container (e.g., a new cartridge of insulin is installed into a pen or pump).

### Virtual Assistant

The dose calculator may be integrated into a virtual assistant - whether standalone (e.g., voice control built into a dose calculator app) or integrated into a 3^{rd} party virtual assistant (e.g., Siri, Google Assistant, Alexa, Cortana, etc.) so the user does not need to view an application at all. This may be useful for convenience, or out of necessity while driving or in an environment where a phone cannot be used.

Asking "How much insulin for a large breakfast?" would prompt a response with the current dose recommendation. Alternatively the user may specify grams of carbs instead of meal type. The assistant may warn if this meal has already been logged, if a dose has been recently taken, or if BG is low and insulin should not be used.

For a meal dose request, the assistant may follow up with, "Would you like to enter a BG value?" to which the user may state their glucose level to include a BG correction in the recommendation

Asking "Do I need a BG correction now?" or "How's my BG?" would prompt a response with the current BG correction dose.

The assistant may also respond to questions about the user's history such as, "When was my last dose?", "When was my last meal?", "How old is my insulin?", "When does my pen expire?", "Did I already take my lunch dose?" and so forth, prompting a readout of the applicable information from the user's data.

### On-Device Display

A smart insulin pen or other insulin dosing device may contain an integrated display for status, reminders, and recommendations. The settings and/or notification content would be pushed from a smart phone or similar.

The pen could display the current recommended meal and/or correction doses, time until the next meal or other event, last dose that was taken, or alerts of a missed dose or time to take a dose.

For meal doses only, once set up the pen could function standalone, displaying appropriate meal doses at the appropriate time until the dose is taken.

For BG correction dose calculations or other contextual information or alerts, the pen would need a connection to the internet or to a smart phone where it can receive additional information such as real-time BG.

### Therapy Reporting

### Overview

The dose calculator application may generate reports to be reviewed by a physician including dose and glucose history, metrics of compliance and usage, and other data analytics and trending.

### Feedback for Adjusting Parameters

The report may include feedback regarding user settings to aid in fine-tuning parameters for the best glycemic control. This may include reporting how often meals were eaten outside of the set time windows, indicating that the meal times may need adjustment; and reporting on glycemic control (e.g., delta from target BG two hours after a dose) broken down by meal or meal size. This may help highlight recommended doses that need adjustment. For example if the user is typically high after breakfast, a larger breakfast insulin dose may be needed.

Because the estimation of carbs or meal sizes is imprecise and prone to human error, it may not be safest to optimize dose sizes to achieve target BG on average, because there will be a distribution around target with some results being dangerously low if the distribution is wide enough. It may be safer to bias dose recommendations so that on average the user ends up slightly above target, so that in cases where carbs or meal size were over-estimated, they are protected from going too low. A distribution of BG results may be analyzed and set so that, for example, the lower 75^{th} percentile of the distribution is at target BG to reduce the incidence of hypoglycemia.

### Compliance Reporting

Typically dose calculator usage and compliance with the recommendation would be reported, but in the case of a touchless dose calculator, these metrics are inferred by how often the user's dose occurs when a recommendation was displayed and matches (or closely matches) the value recommended. That is the best indication of whether the dose calculator was used and followed, since the user may not physically interact with the dose calculator, other than to passively view the recommendation.

In cases where a dose is not recommended, this should not be logged as a missed dose, as the user was following the calculator's recommendation.

In cases where a dose is not taken but it is unclear whether a meal was eaten (e.g., the dose was "missed") or the dose was simply unnecessary, the BG values from this time period can be assessed retroactively. If BG remains stable and in-range, or is low, then the patient should not be counted as having missed a dose since they did not need one. If BG rises or is high, this indicates that a dose should have been taken, likely after food was eaten, and the user did miss a dose. This can be used in reporting compliance with therapy, and occurrence of missed doses.

A missed dose is defined as an occurrence when a dose was recommended, was not taken, and a poor outcome (high BG) resulted. A non-compliant dose is defined as an occurrence when a dose was taken that differed significantly from any recommended dose or when no dose was recommended, and a poor outcome (high or low BG) resulted.

In some embodiments, a method for calculating a metric for missed-dose noncompliance can include the following. For a given past time period, the method identifies occurrences when a dose was recommended but no dose was taken. For each of these occurrences, the method counts only the instances where BG a set time period after the recommendation was a set threshold above target. This value is the number of missed doses. Noncompliance can be calculated by dividing the number of missed doses by the total number of doses recommended in the time period.

In some embodiments, a method for calculating a metric for disregarded-dose-calculator noncompliance can include the following. For a given past time period, the method identifies occurrences when a dose was taken that did not correspond to a recommendation, either because no recommendation was given at the time, or because the dose differed by more than a set threshold from any recommendation given. For each of these occurrences, the method counts only the instances where BG a set time period after the dose was outside a set threshold around target BG. This value is the number of disregarded-dose-calculator doses. Noncompliance can be calculated by dividing the number of disregarded-dose-calculator doses by the total number of doses taken in the time period.

### Reporting on Fixed Dose and Meal Estimation Therapy

With meal types and/or sizes logged or inferred by a patient's dose size, the physician report can list a histogram or bar graph of the meal sizes eaten by the patient, and the number per day (e.g., 0.95), or the total number relative to the total number of days reported (e.g., 12 lunches / 14 days).

The report may contain graphs of the user's glucose, insulin, and dose calculator usage on a particular day or set of days. When the dose calculator has a specific number of carbs entered, this carb value can be graphed directly. For patients on fixed dose or meal estimation therapy there is no direct carb value to graph, so instead the applicable recommended dose size is graphed.

### Evaluation of Current Therapy Method

In the range of therapies from simple (e.g., fixed dose) to complex (e.g., carb-counting), more precise glycemic control is possible from the more complex methods, so patients who start with one therapy may want to transition to a more complex method when they are comfortable. Conversely, someone struggling with their current method may benefit from transitioning to a simpler method that is less stressful and easier to adhere to. In some cases glycemic control may actually be improved by a simpler therapy, such as for someone who very inaccurately counts carbs, meal estimation may actually provide more accurate dose recommendations for their meals.

It would help patients and physicians select which therapy to use and when it is time to switch if the relative benefits could be quantified - such as the improvement expected if switching to a more or less sophisticated method, or the benefit realized since switching from a previous method.

Glycemic control metrics such as estimated A1C, average BG, time in or out of target BG range, BG or delta from target BG after meals, and frequency of hypoglycemic events would be applicable. After switching therapies, these metrics may be empirically measured and reported, giving the patient a "before and after" comparison of their success on the various therapies. This could be reported as actual values, deltas, or percent improvements.

Implementations of the subject matter and the functional operations described in this patent document can be implemented in various systems, digital electronic circuitry, or in computer software, firmware, or hardware, including the structures disclosed in this specification and their structural equivalents, or in combinations of one or more of them. Implementations of the subject matter described in this specification can be implemented as one or more computer program products, i.e., one or more modules of computer program instructions encoded on a tangible and non-transitory computer readable medium for execution by, or to control the operation of, data processing apparatus. The computer readable medium can be a machine-readable storage device, a machine-readable storage substrate, a memory device, a composition of matter effecting a machine-readable propagated signal, or a combination of one or more of them. The term "data processing unit" or "data processing apparatus" encompasses all apparatus, devices, and machines for processing data, including by way of example a programmable processor, a computer, or multiple processors or computers. The apparatus can include, in addition to hardware, code that creates an execution environment for the computer program in question, e.g., code that constitutes processor firmware, a protocol stack, a database management system, an operating system, or a combination of one or more of them.

A computer program (also known as a program, software, software application, script, or code) can be written in any form of programming language, including compiled or interpreted languages, and it can be deployed in any form, including as a stand-alone program or as a module, component, subroutine, or other unit suitable for use in a computing environment. A computer program does not necessarily correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

The processes and logic flows described in this specification can be performed by one or more programmable processors executing one or more computer programs to perform functions by operating on input data and generating output. The processes and logic flows can also be performed by, and apparatus can also be implemented as, special purpose logic circuitry, e.g., an FPGA (field programmable gate array) or an ASIC (application specific integrated circuit).

Processors suitable for the execution of a computer program include, by way of example, both general and special purpose microprocessors, and any one or more processors of any kind of digital computer. Generally, a processor will receive instructions and data from a read only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Computer readable media suitable for storing computer program instructions and data include all forms of nonvolatile memory, media and memory devices, including by way of example semiconductor memory devices, e.g., EPROM, EEPROM, and flash memory devices. The processor and the memory can be supplemented by, or incorporated in, special purpose logic circuitry.

It is intended that the specification, together with the drawings, be considered exemplary only, where exemplary means an example. As used herein, the singular forms "a", "an" and "the" are intended to include the plural forms as well, unless the context clearly indicates otherwise. Additionally, the use of "or" is intended to include "and/or", unless the context clearly indicates otherwise.

## Claims

1. A method (200) for adjusting an insulin dose size by fixed-dose titration on an injection pen device (10) in wireless communication with a mobile communication device (5), the method (200) comprising:
receiving (210) a first glucose measurement of a patient user of the injection pen device (10) prior to consumption of a meal;
determining (215) a first dose size of insulin to be recommended for administration to the patient user based on consumption of the meal, wherein the determined first dose size of insulin is selected from a predefined insulin amount that corresponds to (i) a meal type, or (ii) the meal type and a meal size of the meal type; wherein the predefined insulin amount is estimated from an amount of carbohydrates estimated from the meal type and the meal size;
presenting (220) to the patient user, via a display on at least one of the injection pen device (10) or the mobile communication device (5), the recommended first dose size of insulin to be administered to the patient user;
receiving (225) a second glucose measurement of the patient user within a predefined time period after consumption of the meal by the patient user;
determining (230) a second dose size of insulin to be recommended for administration to the patient user for correcting the second glucose measurement to be within a target glucose level range;
presenting (235) to the patient user, via the display, the recommended second dose size of insulin to be administered to the patient user,
**characterized in that**
quantifying a blood glucose delta for the meal, wherein the blood glucose delta includes a difference between second blood glucose measurement and the first blood glucose measurement, subtracting an expected effect of the correction dose from the first blood glucose measurement, wherein the expected effect of the correction dose corresponds to the correction dose divided by the insulin-to-carb ration,
repeating the steps for various meals and corresponding glucose measurements and producing a plurality of associated blood glucose delta values,
aggregating the plurality of blood glucose delta over multiple meals of the same associated meal type and/or meal size,
quantifying a success metric based on one of the following: (i) average blood glucose delta, (ii) minimum blood glucose delta, or (iii) lower confidence bound of blood glucose delta,
adjusting the predefined insulin amount associated with the meal type and meal size, when the success metric is beyond a threshold, by calculating a new associated dose by summing the initial associated dose with the success metric divided by the user's insulin-to-carb ratio.

2. The method (200) of claim 1, wherein the meal type is selected from a group consisting of breakfast, lunch, dinner, pre-breakfast snack, pre-lunch snack, pre-dinner snack, and post-dinner snack.

3. The method (200) of claim 1, wherein the meal size is selected from a group consisting of a small meal, a medium size meal, and a large size meal.

4. The method (200) of claim 1, wherein the meal type includes a food category.

5. The method (200) of claim 1, comprising:
prior to the determining (230) the second dose size, prompting a confirmation input that the meal was consumed by the patient user.

6. A system (100) for administering a medicine using a fixed-dose titration protocol, comprising:
an injection pen device (10) including a dose setting mechanism to set a dose of a medicine contained in a medicine cartridge that is to be dispensed by the injection pen device (10), a dispensing mechanism to dispense the medicine according to the set dose, and an electronics unit including a processor, a memory comprising instructions executable by the processor, and a wireless transmitter, the processor of the injection pen device(10) configured to generate dose data associated with a dispensing event of a dose of the medicine dispensed from the injection pen device (10) and time data associated with the dispensing event, and to wirelessly transmit the dose data, wherein the medicine includes insulin,
wherein the injection pen device (10) is in wireless communication with a mobile communication device (5) that includes a data processing unit including a processor and memory to receive and process the dose data, and
wherein the mobile communication device (5) includes a software application program product comprising a non-transitory computer-readable storage medium having instructions, which when executed by the processor of the data processing unit, cause the mobile communication device (5) to determine a recommended one or more fixed-dose sizes of the insulin based on (i) health data, including first glucose level of a patient user of the injection pen device (10) that is measured prior to consumption of a meal and a second glucose level of the patient user that is measured within a predefined time period after consumption of the meal, and (ii) meal data, including a meal type of the meal and a meal size of the meal,
wherein the instructions include a fixed-dose titration instruction set to determine the recommended one or more fixed-dose sizes of insulin, wherein the fixed-dose instruction set, when executed by the processor of the data processing unit, cause the mobile communication device (5) to:
receive, from a glucose monitor in communication with the mobile communication device (5), a first glucose measurement of the patient user prior to consumption of the meal;
determine a first dose size of insulin to be recommended for administration to the patient user based on consumption of the meal, wherein the determined first dose size of insulin is selected from a predefined insulin amount that corresponds to (i) the meal type, or to (ii) the meal type and the meal size of the meal type; wherein the predefined insulin amount is estimated from an amount of carbohydrates estimated from the meal type and the meal size;
present to the patient user, via a display on at least one of the injection pen device (10) or the mobile communication device (5), the recommended first dose size of insulin to be administered to the patient user;
receive, from the glucose monitor, a second glucose measurement of the patient user within a predefined time period after consumption of the meal by the patient user;
determine a second dose size of insulin to be recommended for administration to the patient user for correcting the second glucose measurement to be within a target glucose level range; and
present to the patient user, via the display, the recommended second dose size of insulin to be administered to the patient user,
**characterized in that**
quantify a blood glucose delta for the meal, wherein the blood glucose delta includes a difference between second blood glucose measurement and the first blood glucose measurement, subtracting an expected effect of the correction dose from the first blood glucose measurement, wherein the expected effect of the correction dose corresponds to the correction dose divided by the insulin-to-carb ration,
repeat the steps for various meals and corresponding glucose measurements and produce a plurality of associated blood glucose delta values,
aggregate the plurality of blood glucose delta over multiple meals of the same associated meal type and/or meal size,
quantify a success metric based on one of the following: (i) average blood glucose delta, (ii) minimum blood glucose delta, or (iii) lower confidence bound of blood glucose delta,
adjust the predefined insulin amount associated with the meal type and meal size, when the success metric is beyond a threshold, by calculating a new associated dose by summing the initial associated dose with the success metric divided by the user's insulin-to-carb ratio.

7. The system of claim 6, wherein the meal type is selected from a group consisting of breakfast, lunch, dinner, pre-breakfast snack, pre-lunch snack, pre-dinner snack, and post-dinner snack.

8. The system of claim 6, wherein the meal size is selected from a group consisting of a small meal, a medium size meal, and a large size meal.

9. The system of claim 6, wherein the meal type includes a food category.

10. The system of claim 6, wherein the software application program product includes (i) a data aggregator that obtains the health data and the meal data, (ii) a dose calculator that autonomously determines the recommended one or more fixed-dose sizes of the insulin, and (iii) a user interface generator to produce a user interface on the display of the at least one of the injection pen device (10) or the mobile communication device (5).

## Patentansprüche

1. Verfahren (200) zum Anpassen einer Insulindosisgröße durch Festdosistitration an einer Injektionspenvorrichtung (10) in drahtloser Kommunikation mit einer mobilen Kommunikationsvorrichtung (5), wobei das Verfahren (200) umfasst:
Empfangen (210) einer ersten Glukosemessung eines Patientenbenutzers der Injektionspenvorrichtung (10) vor dem Verzehr einer Mahlzeit;
Bestimmen (215) einer ersten Dosisgröße von Insulin, die zur Verabreichung an den Patientenbenutzer basierend auf dem Verzehr der Mahlzeit empfohlen werden soll, wobei die bestimmte erste Dosisgröße von Insulin aus einer vordefinierten Insulinmenge ausgewählt wird, die (i) einer Mahlzeitenart oder (ii) der Mahlzeitenart und einer Mahlzeitengröße der Mahlzeitenart entspricht; wobei die vordefinierte Insulinmenge aus einer Kohlenhydratmenge geschätzt wird, die aus der Mahlzeitenart und der Mahlzeitengröße geschätzt wird;
Präsentieren (220), dem Patientenbenutzer, der empfohlenen ersten Dosisgröße von Insulin, die dem Patientenbenutzer verabreicht werden soll, mittels einer Anzeige auf mindestens einer der Injektionspenvorrichtung (10) oder der mobilen Kommunikationsvorrichtung (5);
Empfangen (225) einer zweiten Glukosemessung des Patientenbenutzers innerhalb einer vordefinierten Zeitspanne nach dem Verzehr der Mahlzeit durch den Patientenbenutzer;
Bestimmen (230) einer zweiten Dosisgröße von Insulin, die zur Verabreichung an den Patientenbenutzer empfohlen werden soll, zum Korrigieren der zweiten Glukosemessung, um innerhalb eines Ziel-Glukosespiegelbereichs zu liegen;
Präsentieren (235), dem Patientenbenutzer, der empfohlenen zweiten Dosisgröße von Insulin, die dem Patientenbenutzer verabreicht werden soll, mittels der Anzeige,
**dadurch gekennzeichnet, dass**
Quantifizieren eines Blutglukose-Deltas für die Mahlzeit, wobei das Blutglukose-Delta eine Differenz zwischen der zweiten Blutglukosemessung und der ersten Blutglukosemessung einschließt, Subtrahieren einer erwarteten Wirkung der Korrekturdosis von der ersten Blutglukosemessung, wobei die erwartete Wirkung der Korrekturdosis der Korrekturdosis geteilt durch das Insulin-Kohlenhydrat-Verhältnis entspricht,
Wiederholen der Schritte für verschiedene Mahlzeiten und entsprechende Glukosemessungen und Erzeugen einer Vielzahl von assoziierten Blutglukose-Deltawerten,
Aggregieren der Vielzahl von Blutglukose-Deltas über mehrere Mahlzeiten der gleichen assoziierten Mahlzeitenart und/oder Mahlzeitengröße,
Quantifizieren einer Erfolgsmetrik basierend auf einem der Folgenden: (i) durchschnittliches Blutglukose-Delta, (ii) minimales Blutglukose-Delta oder (iii) untere Konfidenzgrenze des Blutglukose-Deltas,
Anpassen der vordefinierten Insulinmenge, die mit der Mahlzeitenart und der Mahlzeitengröße assoziiert ist, wenn die Erfolgsmetrik über einem Schwellenwert liegt, durch Berechnen einer neuen assoziierten Dosis durch Summieren der anfänglichen assoziierten Dosis mit der Erfolgsmetrik, geteilt durch das Insulin-Kohlenhydrat-Verhältnis des Benutzers.

2. Verfahren (200) nach Anspruch 1, wobei die Mahlzeitenart ausgewählt wird aus einer Gruppe bestehend aus Frühstück, Mittagessen, Abendessen, Snack vor dem Frühstück, Snack vor dem Mittagessen, Snack vor dem Abendessen und Snack nach dem Abendessen.

3. Verfahren (200) nach Anspruch 1, wobei die Mahlzeitengröße ausgewählt wird aus einer Gruppe bestehend aus einer kleinen Mahlzeit, einer Mahlzeit mittlerer Größe und einer Mahlzeit großer Größe.

4. Verfahren (200) nach Anspruch 1, wobei die Mahlzeitenart eine Lebensmittelkategorie einschließt.

5. Verfahren (200) nach Anspruch 1, umfassend:
vor dem Bestimmen (230) der zweiten Dosisgröße, Anfordern einer Bestätigungseingabe, dass die Mahlzeit durch den Patientenbenutzer verzehrt wurde.

6. System (100) zum Verabreichen eines Medikaments unter Verwendung eines Festdosistitrationsprotokolls, umfassend:
eine Injektionspenvorrichtung (10), die einen Dosiseinstellmechanismus zum Einstellen einer Dosis eines in einer Medikamentenkartusche enthaltenen Medikaments, das durch die Injektionspenvorrichtung (10) abgegeben werden soll, einen Abgabemechanismus zum Abgeben des Medikaments gemäß der eingestellten Dosis und eine Elektronikeinheit einschließt, die einen Prozessor, einen Speicher, der durch den Prozessor ausführbare Anweisungen umfasst, und einen drahtlosen Sender einschließt, wobei der Prozessor der Injektionspenvorrichtung(10) konfiguriert ist, um Dosisdaten, die mit einem Abgabeereignis einer Dosis des aus der Injektionspenvorrichtung (10) abgegebenen Medikaments assoziiert sind, und Zeitdaten, die mit dem Abgabeereignis assoziiert sind, zu erzeugen und die Dosisdaten drahtlos zu übertragen, wobei das Medikament Insulin einschließt,
wobei die Injektionspenvorrichtung (10) in drahtloser Kommunikation mit einer mobilen Kommunikationsvorrichtung (5) vorliegt, die eine Datenverarbeitungseinheit einschließt, die einen Prozessor und Speicher einschließt, um die Dosisdaten zu empfangen und zu verarbeiten, und
wobei die mobile Kommunikationsvorrichtung (5) ein Softwareanwendungsprogrammprodukt einschließt, das ein nichtflüchtiges computerlesbares Speichermedium mit Anweisungen umfasst, die, wenn durch den Prozessor der Datenverarbeitungseinheit ausgeführt, bewirken, dass die mobile Kommunikationsvorrichtung (5) eine oder mehrere empfohlene Festdosisgrößen des Insulins bestimmt, basierend auf (i) Gesundheitsdaten, einschließlich eines ersten Glukosespiegels eines Patientenbenutzers der Injektionspenvorrichtung (10), der vor dem Verzehr einer Mahlzeit gemessen wird, und eines zweiten Glukosespiegels des Patientenbenutzers, der innerhalb einer vordefinierten Zeitspanne nach dem Verzehr der Mahlzeit gemessen wird, und (ii) Mahlzeitendaten, einschließlich einer Mahlzeitenart der Mahlzeit und einer Mahlzeitengröße der Mahlzeit,
wobei die Anweisungen einen Festdosistitrationsanweisungssatz einschließen, um die empfohlene eine oder mehreren Festdosisgrößen von Insulin zu bestimmen, wobei der Festdosisanweisungssatz, wenn durch den Prozessor der Datenverarbeitungseinheit ausgeführt, bewirkt, dass die mobile Kommunikationsvorrichtung (5):
von einem Glukosemonitor in Kommunikation mit dem mobilen Kommunikationsgerät (5) eine erste Glukosemessung des Patientenbenutzers vor dem Verzehr der Mahlzeit empfängt;
eine erste Dosisgröße von Insulin bestimmt, die zur Verabreichung an den Patientenbenutzer basierend auf dem Verzehr der Mahlzeit empfohlen werden soll, wobei die bestimmte erste Dosisgröße von Insulin aus einer vorbestimmten Insulinmenge ausgewählt ist, die (i) der Mahlzeitenart oder (ii) der Mahlzeitenart und der Mahlzeitengröße der Mahlzeitenart entspricht; wobei die vordefinierte Insulinmenge aus einer Kohlenhydratmenge geschätzt wird, die aus der Mahlzeitenart und der Mahlzeitengröße geschätzt wird;
die empfohlene erste Dosisgröße von Insulin, die dem Patientenbenutzer verabreicht werden soll, dem Patientenbenutzer mittels einer Anzeige auf mindestens einer der Injektionspenvorrichtung (10) oder der mobilen Kommunikationsvorrichtung (5) präsentiert;
eine zweite Glukosemessung des Patientenbenutzers innerhalb einer vordefinierten Zeitspanne nach dem Verzehr der Mahlzeit durch den Patientenbenutzer empfängt;
eine zweite Dosisgröße von Insulin bestimmt, die zur Verabreichung an den Patientenbenutzer empfohlen werden soll, zum Korrigieren der zweiten Glukosemessung, um innerhalb eines Ziel-Glukosespiegelbereichs zu liegen; und
die empfohlene zweite Dosisgröße von Insulin, die dem Patientenbenutzer verabreicht werden soll, dem Patientenbenutzer mittels der Anzeige präsentiert,
**dadurch gekennzeichnet, dass**
ein Blutglukose-Delta für die Mahlzeit quantifiziert, wobei das Blutglukose-Delta eine Differenz zwischen der zweiten Blutglukosemessung und der ersten Blutglukosemessung einschließt, eine erwartete Wirkung der Korrekturdosis von der ersten Blutglukosemessung subtrahiert, wobei die erwartete Wirkung der Korrekturdosis der Korrekturdosis geteilt durch das Insulin-Kohlenhydrat-Verhältnis entspricht,
die Schritte für verschiedene Mahlzeiten und entsprechende Glukosemessungen wiederholt und eine Vielzahl von assoziierten Blutglukose-Deltawerten erzeugt,
die Vielzahl von Blutglukose-Deltas über mehrere Mahlzeiten der gleichen assoziierten Mahlzeitenart und/oder Mahlzeitengröße aggregiert,
eine Erfolgsmetrik basierend auf einem der Folgenden quantifiziert: (i) durchschnittliches Blutglukose-Delta, (ii) minimales Blutglukose-Delta oder (iii) untere Konfidenzgrenze des Blutglukose-Deltas,
die vordefinierte Insulinmenge, die mit der Mahlzeitenart und der Mahlzeitengröße assoziiert ist, wenn die Erfolgsmetrik über einem Schwellenwert liegt, durch Berechnen einer neuen assoziierten Dosis durch Summieren der anfänglichen assoziierten Dosis mit der Erfolgsmetrik, geteilt durch das Insulin-Kohlenhydrat-Verhältnis des Benutzers anpasst.

7. System nach Anspruch 6, wobei die Mahlzeitenart ausgewählt ist aus einer Gruppe bestehend aus Frühstück, Mittagessen, Abendessen, Snack vor dem Frühstück, Snack vor dem Mittagessen, Snack vor dem Abendessen und Snack nach dem Abendessen.

8. System nach Anspruch 6, wobei die Mahlzeitengröße ausgewählt ist aus einer Gruppe bestehend aus einer kleinen Mahlzeit, einer Mahlzeit mittlerer Größe und einer Mahlzeit großer Größe.

9. System nach Anspruch 6, wobei die Mahlzeitenart eine Lebensmittelkategorie einschließt.

10. System nach Anspruch 6, wobei das Softwareanwendungsprogrammprodukt (i) einen Datenaggregator, der die Gesundheitsdaten und die Mahlzeitendaten erhält, (ii) einen Dosisrechner, der die eine oder die mehreren empfohlenen Festdosisgrößen des Insulins autonom bestimmt, und (iii) einen Bedienerschnittstellengenerator einschließt, um eine Bedienerschnittstelle auf der Anzeige der mindestens einen der Injektionspenvorrichtung (10) oder der mobilen Kommunikationsvorrichtung (5) zu erzeugen.

## Revendications

1. Procédé (200) d'ajustement d'une taille de dose d'insuline par titrage à dose fixe sur un dispositif de stylo d'injection (10) en communication sans fil avec un dispositif de communication mobile (5), le procédé (200) comprenant :
la réception (210) d'une première mesure de glucose d'un patient utilisateur du dispositif de stylo d'injection (10) avant la consommation d'un repas ;
la détermination (215) d'une première taille de dose d'insuline à recommander pour une administration au patient utilisateur en fonction de la consommation du repas, dans lequel la première taille de dose d'insuline déterminée est choisie à partir d'une quantité d'insuline prédéfinie qui correspond à (i) un type de repas, ou (ii) au type de repas et à une taille de repas du type de repas ; dans lequel la quantité d'insuline prédéfinie est estimée à partir d'une quantité de glucides estimée à partir du type de repas et de la taille du repas ;
la présentation (220) au patient utilisateur, par l'intermédiaire d'un affichage sur au moins l'un parmi le dispositif de stylo d'injection (10) ou le dispositif de communication mobile (5), de la première taille de dose d'insuline recommandée à administrer au patient utilisateur ;
la réception (225) d'une seconde mesure de glucose du patient utilisateur dans un laps de temps prédéfini après la consommation du repas par le patient utilisateur ;
la détermination (230) d'une seconde taille de dose d'insuline à recommander pour une administration au patient utilisateur pour corriger la seconde mesure de glucose afin qu'elle soit comprise dans une plage de taux de glucose cible ;
la présentation (235) au patient utilisateur, par l'intermédiaire de l'affichage, de la taille recommandée de la seconde dose d'insuline à administrer au patient utilisateur,
**caractérisé en ce que**
la quantification d'un delta de glucose sanguin pour le repas, dans lequel le delta de glucose sanguin comporte une différence entre la seconde mesure de glucose sanguin et la première mesure de glucose sanguin, la soustraction d'un effet attendu de la dose de correction de la première mesure de glucose sanguin, dans lequel l'effet attendu de la dose de correction correspond à la dose de correction divisée par le ratio insuline-glucides,
la répétition des étapes pour divers repas et des mesures de glucose correspondantes et la production d'une pluralité de valeurs delta de glucose sanguin associées,
l'agrégation de la pluralité de deltas de glucose sanguin sur plusieurs repas du même type de repas associé et/ou de la taille de repas,
la quantification d'une métrique de succès en fonction de l'un parmi ce qui suit : (i) delta moyen de glucose sanguin, (ii) delta minimum de glucose sanguin, ou (iii) limite de confiance inférieure du delta de glucose sanguin,
l'ajustement de la quantité d'insuline prédéfinie associée au type de repas et à la taille du repas, lorsque la métrique de réussite est au-delà d'un seuil, en calculant une nouvelle dose associée en additionnant la dose associée initiale avec la métrique de réussite divisée par le ratio insuline-glucides de l'utilisateur.

2. Procédé (200) selon la revendication 1, dans lequel le type de repas est choisi parmi un groupe constitué par un petit-déjeuner, un déjeuner, un dîner, une collation avant le petit-déjeuner, une collation avant le déjeuner, une collation avant le dîner et une collation après le dîner.

3. Procédé (200) selon la revendication 1, dans lequel la taille du repas est choisie parmi un groupe constitué par un petit repas, un repas de taille moyenne et un repas de grande taille.

4. Procédé (200) selon la revendication 1, dans lequel le type de repas comporte une catégorie d'aliments.

5. Procédé (200) selon la revendication 1, comprenant :
avant la détermination (230) de la taille de la seconde dose, la demande d'une entrée de confirmation selon laquelle le repas a été consommé par le patient utilisateur.

6. Système (100) d'administration d'un médicament à l'aide d'un protocole titrage à dose fixe, comprenant :
un dispositif de stylo d'injection (10) incluant un mécanisme de réglage de dose pour régler une dose d'un médicament contenu dans une cartouche de médicament qui doit être distribué par le dispositif de stylo d'injection (10), un mécanisme de distribution pour distribuer le médicament selon la dose réglée, et une unité électronique incluant un processeur, une mémoire comprenant des instructions exécutables par le processeur, et un émetteur sans fil, le processeur du dispositif de stylo d'injection (10) étant configuré pour générer des données de dose associées à un événement de distribution d'une dose du médicament distribué à partir du dispositif de stylo d'injection (10) et des données temporelles associées à l'événement de distribution, et pour transmettre sans fil les données de dose, dans lequel le médicament comporte de l'insuline,
dans lequel le dispositif de stylo d'injection (10) est en communication sans fil avec un dispositif de communication mobile (5) qui comporte une unité de traitement de données incluant un processeur et une mémoire pour recevoir et traiter les données de dose, et
dans lequel le dispositif de communication mobile (5) comporte un produit de programme d'application logiciel comprenant un support de stockage non transitoire lisible par ordinateur ayant des instructions qui, lorsqu'elles sont exécutées par le processeur de l'unité de traitement de données, amènent le dispositif de communication mobile (5) à déterminer une ou plusieurs tailles de dose fixe recommandées de l'insuline en fonction (i) de données de santé, incluant un premier niveau de glucose d'un patient utilisateur du dispositif de stylo d'injection (10) qui est mesuré avant la consommation d'un repas et un second niveau de glucose du patient utilisateur qui est mesuré dans un laps temps prédéfini après la consommation du repas, et (ii) de données de repas, incluant un type de repas du repas et une taille de repas du repas,
dans lequel les instructions comportent un ensemble d'instructions de titrage à dose fixe pour déterminer la ou les tailles de dose fixe recommandées d'insuline, dans lequel l'ensemble d'instructions à dose fixe, lorsqu'elles sont exécutées par le processeur de l'unité de traitement de données, amènent le dispositif de communication mobile (5) à :
recevoir, à partir d'un moniteur de glucose en communication avec le dispositif de communication mobile (5), une première mesure de glucose du patient utilisateur avant la consommation du repas ;
déterminer une première taille de dose d'insuline à recommander pour une administration au patient utilisateur en fonction de la consommation du repas, dans lequel la première taille de dose d'insuline déterminée est sélectionnée à partir d'une quantité d'insuline prédéfinie qui correspond (i) au type de repas, ou (ii) au type de repas et à la taille de repas du type de repas ; dans lequel la quantité d'insuline prédéfinie est estimée à partir d'une quantité de glucides estimée à partir du type de repas et de la taille du repas ;
présenter au patient utilisateur, par l'intermédiaire d'un affichage sur au moins l'un parmi le dispositif de stylo d'injection (10) ou le dispositif de communication mobile (5), la première taille de dose d'insuline recommandée à administrer au patient utilisateur ;
recevoir, à partir du moniteur de glucose, une seconde mesure de glucose du patient utilisateur dans un laps de temps prédéfini après la consommation du repas par le patient utilisateur ;
déterminer une seconde taille de dose d'insuline à recommander pour une administration au patient utilisateur pour corriger la seconde mesure de glucose afin qu'elle se situe dans une plage de taux de glucose cible ; et
présenter au patient utilisateur, par l'intermédiaire de l'affichage, la taille recommandée de la seconde dose d'insuline à administrer au patient utilisateur,
**caractérisé en ce que**
quantifier un delta de glucose sanguin pour le repas, dans lequel le delta de glucose sanguin comporte une différence entre la seconde mesure de glucose sanguin et la première mesure de glucose sanguin, soustraire un effet attendu de la dose de correction de la première mesure de glucose sanguin, dans lequel l'effet attendu de la dose de correction correspond à la dose de correction divisée par le ratio insuline-glucides,
répéter les étapes pour divers repas et les mesures de glucose correspondantes et la production d'une pluralité de valeurs delta de glucose sanguin associées,
agréger la pluralité de deltas de glucose sanguin sur plusieurs repas du même type de repas associé et/ou de la même taille de repas,
quantifier une métrique de succès en fonction de l'un parmi ce qui suit : (i) delta moyen de glucose sanguin, (ii) delta minimum de glucose sanguin, ou (iii) limite de confiance inférieure du delta de glucose sanguin,
ajuster la quantité d'insuline prédéfinie associée au type de repas et à la taille du repas, lorsque la métrique de réussite est au-delà d'un seuil, en calculant une nouvelle dose associée en additionnant la dose associée initiale avec la métrique de réussite divisée par le ratio insuline-glucides de l'utilisateur.

7. Système selon la revendication 6, dans lequel le type de repas est choisi parmi un groupe constitué par un petit-déjeuner, un déjeuner, un dîner, une collation avant le petit-déjeuner, une collation avant le déjeuner, une collation avant le dîner et une collation après le dîner.

8. Système selon la revendication 6, dans lequel la taille du repas est choisie parmi un groupe constitué par un petit repas, un repas de taille moyenne et un repas de grande taille.

9. Système selon la revendication 6, dans lequel le type de repas comporte une catégorie d'aliments.

10. Système selon la revendication 6, dans lequel le produit de programme d'application logiciel comporte (i) un agrégateur de données qui obtient les données de santé et les données de repas, (ii) un calculateur de dose qui détermine de manière autonome la ou les tailles de dose fixe recommandées de l'insuline, et (iii) un générateur d'interface utilisateur pour produire une interface utilisateur sur l'affichage de l'au moins l'un parmi le dispositif de stylo d'injection (10) ou le dispositif de communication mobile (5).
